(19) Europäisches Patentamt
European Patent Office
Office européen des brevets

(11) Veröffentlichungsnummer : **0 490 820 A2**

(12) # EUROPÄISCHE PATENTANMELDUNG

(21) Anmeldenummer : **91810945.5**

(51) Int. Cl.[5] : **C07D 257/04,** A61K 31/41

(22) Anmeldetag : **05.12.91**

(30) Priorität : **14.12.90 CH 3965/90**

(43) Veröffentlichungstag der Anmeldung :
**17.06.92 Patentblatt 92/25**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Schmidlin, Tibur, Dr.**
**Friedensgasse 36**
**CH-4056 Basel (CH)**
Erfinder : **Ostermayer, Franz, Dr.**
**Am Hang 5**
**CH-4125 Riehen (CH)**
Erfinder : **Bühlmayer, Peter, Dr.**
**Hangstrasse 18**
**CH-4144 Arlesheim (CH)**

(54) **Biphenylyl-Verbindungen.**

(57) Biphenylyl-Verbindungen der Formel

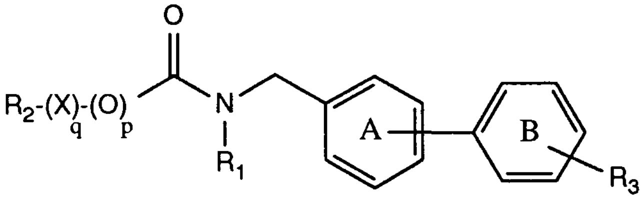

(I),

worin die Variablen $R_1$, $R_2$, $R_3$, p, q und X und die Ringe A und B die in Anspruch 1 angegebenen Bedeutungen besitzen, in freier Form oder in Salzform, sind in an sich bekannter Weise herstellbar und können beispielsweise als Arzneimittelwirkstoffe verwendet werden.

EP 0 490 820 A2

Jouve, 18, rue Saint-Denis, 75001 PARIS

Die Erfindung betrifft Biphenylyl-Verbindungen der Formel

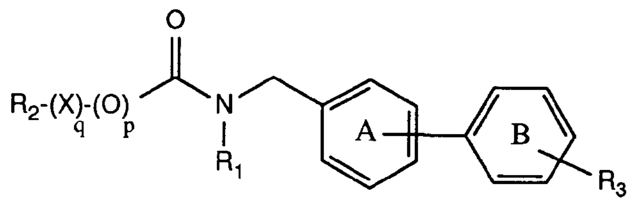

(I),

worin $R_1$ ein aliphatischer Kohlenwasserstoffrest, der gebebenenfalls in höherer als der β-Stellung zu dem in der Formel I gezeigten Stickstoffatom durch Halogen oder Hydroxy substituiert ist, ein cycloaliphatischer Kohlenwasserstoffrest oder ein araliphatischer Kohlenwasserstoffrest ist; $R_2$ 1H-Tetrazol-5-yl, Carboxy, verestertes Carboxy, amidiertes Carboxy, Amino, substituiertes Amino, Acylamino, Formyl, acetalisiertes Formyl, Hydroxy, verethertes Hydroxy, $S(O)_m$-R, wobei m 0, 1 oder 2 ist und R Wasserstoff oder ein aliphatischer Kohlenwasserstoffrest ist, Alkanoyl, Aminosulfonyl, N-substituiertes Aminosulfonyl, $PO_2H_2$, $PO_3H_2$ oder S-substituiertes Sulfonylamino bedeutet; $R_3$ Carboxy, 1H-Tetrazol-5-yl, $SO_3H$, $PO_2H_2$, $PO_3H_2$ oder Halogenalkansulfonylamino ist; X geradkettiges Alkylen, das gegebenenfalls aliphatisch überbrückt und/oder gegebenenfalls durch einen aliphatischen Kohlenwasserstoffrest substituiert ist, darstellt, oder für das Strukturelement der Formel -$X_1$-Ph-$X_2$- steht, wobei $X_1$ und $X_2$ unabhängig voneinander für eine Bindung oder für Alkylen stehen und Ph gegebenenfalls substituiertes Phenylen bedeutet; die Ringe A und B unabhänbig voneinander unsubstituiert oder durch Halogen, Hydroxy, verethertes Hydroxy, $S(O)_m$-R, wobei m 0, 1 oder 2 ist und R Wasserstoff oder ein aliphatischer Kohlenwasserstoffrest ist, oder einen gegebenenfalls durch O unterbrochenen und/oder gebebenenfalls durch Halogen oder Hydroxy substituierten aliphatischen Kohlenwasserstoffrest substituiert sind und entweder p und q beide für 1 stehen oder p 0 ist und q 0 oder 1 darstellt; in freier Form oder in Salzform; die Verwendung dieser Verbindungen, ein Verfahren zur Herstellung dieser Verbindungen und pharmazeutische Präparate, enthaltend, in freier Form oder in Form eines pharmazeutisch verwendbaren Salzes, eine solche Verbindung I.

Die Verbindungen I können als, insbesondere pharmazeutisch verwendbare, Salze vorliegen. Weisen die Verbindungen I mindestens ein basisches Zentrum auf, können sie Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten $C_1$-$C_4$-Alkancarbonsäuren, z.B. Essigsäure, wie gegebenenfalls ungesättigten Dicarbonsäuren, z.B. Oxal-, Malon-, Bernstein-, Malein-, Fumar-, Phthal- oder Terephthalsäure, wie Hydroxycarbonsäuren, z.B. Ascorbin-, Glykol-, Milch-, Äpfel-, Wein- oder Zitronensäure, wie Aminosäuren, z.B. Asparagin- oder Glutaminsäure, oder wie Benzoesäure, oder mit organischen Sulfonsäuren, wie gegebenenfalls, z.B. durch Halogen, substituierten $C_1$-$C_4$-Alkan- oder Aryl-sulfonsäuren, z.B. Methan- oder p-Toluolsulfonsäure, gebildet. Entsprechende Säureadditionssalze können auch mit einem gegebenenfalls zusätzlich vorhandenen basischen Zentrum gebildet werden. Femer können Verbindungen I mit mindestens einer aciden Gruppe (beispielsweise COOH oder 1H-Tetrazol-5-yl) Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Thiomorpholin, Piperidin, Pyrrolidin, einem Mono-, Di- oder Triniederalkylamin, z. B. Ethyl-, tert.-Butyl-, Diethyl-, Diisopropyl-, Triethyl-, Tributyl- oder Dimethyl-propyl-amin, oder einem Mono-, Di- oder Trihydroxyniederalkylamin, z.B. Mono-, Di- oder Triethanolamin. Weiterhin können gegebenenfalls entsprechende innere Salze gebildet werden. Umfasst sind ferner für pharmazeutische Verwendungen nicht geeignete Salze, die beispielsweise für die Isolierung bzw. Reinigung von freien Verbindungen I oder deren pharmazeutisch verwendbaren Salzen eingesetzt werden können.

Die Ringe A und B bilden einen Biphenylylrest, wobei entsprechendes Biphenyl-4-yl bevorzugt ist und der Rest $R_3$ vorzugsweise in einer 2-Position von Ring B lokalisiert ist. Die Rinbe A und B sind unabhängig voneinander unsubstituiert oder ein- oder mehrfach, z.B. zwei- oder dreifach, substituiert, wobei die Substituenten aus der Gruppe der entsprechenden vorstehend aufgeführten Substituenten ausgewählt sein können.

Ein aliphatischer Kohlenwasserstoffrest ist Niederalkyl, Niederalkenyl oder in zweiter Linie Niederalkinyl.

Ein durch Halogen oder Hydroxy substituierter aliphatischer Kohlenwasserstoffrest ist Halogen-niederalkyl, -niederalkenyl oder -niederalkinyl oder Hydroxy-niederalkyl, -niederalkenyl oder -niederalkinyl.

Ein aliphatischer Kohlenwasserstoffrest, der in höherer als der β-Stellung zu dem in der Formel I gezeigten Stickstoffatom durch Halogen oder Hydroxy substituiert ist, ist ein vorstehend definierter durch Halogen oder Hydroxy substituierter aliphatischer Kohlenwasserstoffrest, in welchem jedoch die Kohlenstoffatome (C-Atome) in α- und β-Stellung zu dem in der Formel I gezeigten Stickstoffatom unsubstituiert sind.

Ein aliphatischer Kohlenwasserstoffrest, der durch O unterbrochen ist, ist Niederalkoxy-niederalkyl, -niederalkenyl oder -niederalkinyl oder Niederalkenyloxy-niederalkyl, -niederalkenyl oder -niederalkinyl.

Ein durch Halogen oder Hydroxy substituierter und durch O unterbrochener aliphatischer Kohlenwasserstoffrest ist ein vorstehend definierter durch O unterbrochener aliphatischer Kohlenwasserstoffrest, der durch Halogen oder Hydroxy substituiert ist.

Ein cycloaliphatischer Kohlenwasserstoffrest ist Cycloalkyl oder in zweiter Linie Cycloalkenyl.

Ein araliphatischer Kohlenwasserstoffrest ist Phenylniederalkyl oder in zweiter Linie Phenyl-niederalkenyl oder -niederalkinyl.

Geradkettiges Alkylen, das aliphatisch überbrückt ist, bedeutet geradkettiges Alkylen, in welchem eine oder unabhängig voneinander zwei Methylengruppe(n) durch Cycloalk-1,1-ylen ersetzt ist (sind).

Geradkettiges Alkylen, das durch einen aliphatischen Kohlenwasserstoffrest substituiert ist, bedeutet geradkettiges Alkylen, das ein- oder unabhänbig voneinander zwei-, drei- oder vierfach durch Niederalkyl, Niederalkenyl oder in zweiter Linie Niederalkinyl substituiert ist.

Geradkettiges Alkylen, das überbrückt und durch einen aliphatischen Kohlenwasserstoffrest substituiert ist, ist vorstehend definiertes durch einen aliphatischen Kohlenwasserstoffrest substituiertes geradkettiges Alkylen, in welchem eine oder unabhängig voneinander zwei Methylengruppe(n) der in der entsprechenden unsubstituierten geradkettigen Alkylengruppe vorhandenen Methylengruppen durch Cycloalk-1,1-ylen ersetzt ist (sind).

Verestertes Carboxy ist Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist.

Amidiertes Carboxy ist Carbamoyl, dessen Aminogruppe unsubstituiert oder durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist.

Substituiertes Amino ist durch Niederalkyl, Niederalkenyl, Niederalkinyl oder Phenyl-niederalkyl, -niederalkenyl oder -niederalkinyl mono- oder unabhängig voneinander disubstituiertes oder durch Niederalkylen oder Niederalkylenoxyniederalkylen disubstituiertes Amino.

Acylamino ist durch Niederalkanoyl oder Benzoyl monosubstituiertes Amino.

Acetalisiertes Formyl ist Diniederalkoxymethyl oder 1,3-Dioxacycloalk-2-yl.

Verethertes Hydroxy ist Niederalkoxy, Niederalkenyloxy, Phenoxy oder Benzyloxy.

Alkanoyl bedeutet Niederalkanoyl.

N-substituiertes Aminosulfonyl ist Aminosulfonyl, in welchem die Aminogruppe durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist.

S-substituiertes Sulfonylamino ist Sulfonylamino, in welchem das Schwefelatom der Sulfonylaminogruppe durch Niederalkyl, Niederalkenyl, Niederalkinyl, Halogen-niederalkyl, -niederalkenyl oder -niederalkinyl, Phenyl-niederalkyl, -niederalkenyl oder -niederalkinyl oder Phenyl monosubstituiert ist.

Vor- und nachstehend bedeutet Phenyl jeweils unsubstituiertes oder ein- oder unabhängig voneinander mehrfach, z.B. zwei- oder dreifach, z.B. durch (einen) Substituenten ausgewählt aus der Gruppe, die aus Halogen, Hydroxy, Niederalkoxy, Niederalkyl und Trifluormethyl besteht, substituiertes Phenyl. Dies gilt für Phenylreste per se und für Phenylteilstrukturen in allen Phenylteilstrukturen enthaltenden Gruppen, d. h. in Phenylen-, Phenylniederalkyl-, Phenylniederalkenyl-, Phenylniederalkinyl-, Benzoyl-, Phenoxy- und Benzyloxy-Gruppen.

Die vor- und nachstehend verwendeten Allgemeinbegriffe haben, sofern nicht abweichend definiert, die nachfolgend angegebenen Bedeutungen.

Der Ausdruck " Nieder" bedeutet, dass entsprechende Gruppen und Verbindungen jeweils 1 bis und mit 7, vorzugsweise 1 bis und mit 4, C-Atome enthalten.

Halogen ist insbesondere Halogen mit einer Atomnummer bis und mit 35, d. h. Fluor, Chlor oder Brom, und umfasst ferner Iod.

Geradkettiges Alkylen ist geradkettiges Niederalkylen, d. h. geradkettiges $C_1$-$C_7$-Alkylen, d. h. Methylen, Eth-1,2-ylen, Prop-1,3-ylen, But-1,4-ylen, Pent-1,5-ylen, Hex-1,6-ylen oder Hept-1,7-ylen, wobei die Numerierung der C-Atome dieser geradkettigen Alkylengruppen in der Weise erfolgt, dass das in der Formel I der Gruppe $(O)_p$ benachbarte C-Atom die Nummer 1 trägt.

Halogenalkansulfonylamino bedeutet Halogenniederalkansulfonylamino, d. h. Halogen-$C_1$-$C_7$-alkansulfonylamino, und ist z.B. Trifluormethan-, Difluormethan-, 1, 1,2-Trifluorethan- oder Heptafluorpropan-sulfonylamino. Bevorzugt ist Halogen-$C_1$-$C_4$-alkansulfonylamino.

Phenylen ist 1,2-, 1,3- oder 1,4-Phenylen

Alkylen ($X_1$ bzw. $X_2$) ist insbesondere $C_1$-$C_7$-Alkylen, ist geradkettig oder verzweigt und bedeutet insbesondere Methylen, Eth-1,2-ylen, Prop-1,3-ylen, But-1,4-ylen, Pent-1,5-ylen, Prop-1,2-ylen, 2-Methylprop-1,3-

ylen oder 2,2-Dimethylprop-1,3-ylen. Bevorzugt ist geradkettiges $C_1$-$C_3$-Alkylen.

Niederalkyl ist $C_1$-$C_7$-Alkyl, d. h. Methyl, Ethyl, n-Propyl, Isopropyl, n-Butyl, Isobutyl, sek.-Butyl, tert.-Butyl oder ein entsprechender Pentyl-, Hexyl- oder Heptylrest. Bevorzugt ist $C_1$-$C_4$-Alkyl.

Niederalkenyl bedeutet $C_3$-$C_7$-Alkenyl und ist z.B. Propen-2-yl, Allyl oder But-1-en-3-yl, -1-en-4-yl, -2-en-1-yl oder -2-en-2-yl. Bevorzugt ist $C_3$-$C_5$-Alkenyl.

Niederalkinyl ist $C_3$-$C_7$-Alkinyl und bedeutet z. B. Propargyl.

Halogenniederalkyl bedeutet insbesondere Halogen-$C_1$-$C_4$-alkyl, wie Trifluormethyl, 2-Chlor-1,1,2-trifluorethyl, Chlormethyl, 3,3,3-Trifluorpropyl, 4-Chlorbutyl oder Heptafluorpropyl.

Halogenniederalkenyl bedeutet insbesondere Halogen-$C_3$-$C_5$-alkenyl, wie 3-Chlorallyl.

Halogenniederalkinyl ist insbesondere Halogen-$C_3$-$C_5$-alkinyl, wie 3-Chlorpropargyl.

Hydroxyniederalkyl bedeutet insbesondere Hydroxy-$C_1$-$C_4$-alkyl, wie Hydroxymethyl, 2-Hydroxyethyl, 3-Hydroxypropyl oder 4-Hydroxybutyl.

Hydroxyniederalkenyl bedeutet insbesondere Hydroxy-$C_3$-$C_5$-alkenyl, wie 3-Hydroxyallyl.

Hydroxyniederalkinyl bedeutet insbesondere Hydroxy-$C_3$-$C_5$-alkinyl, wie 3-Hydroxypropargyl.

Niederalkoxyniederalkyl bedeutet $C_1$-$C_4$-Alkoxy-$C_1$-$C_4$-alkyl, wie 2-Methoxyethyl, 2-Ethoxyethyl, 2-(n-Propyloxy)ethyl oder Ethoxymethyl.

Niederalkoxy-niederalkenyl bzw. -niederalkinyl bedeutet $C_1$-$C_4$-Alkoxy-$C_3$-$C_5$-alkenyl bzw. -alkinyl.

Niederalkenyloxyniederalkyl bedeutet $C_3$-$C_5$-Alkenyloxy-$C_1$-$C_4$-alkyl, wie 2-Allyloxyethyl, und Niederalkenyloxy-niederalkenyl bzw. -niederalkinyl bedeutet $C_3$-$C_5$-Alkenyloxy-$C_3$-$C_5$-alkenyl bzw. -alkinyl.

Cycloalkyl ist Cycloniederalkyl, d. h. $C_3$-$C_7$-Cycloalkyl, d. h. Cyclopropyl, Cyclobutyl, Cyclopentyl, Cyclohexyl oder Cycloheptyl. Bevorzugt sind Cyclopentyl und Cyclohexyl.

Cycloalkenyl ist Cycloniederalkenyl, d. h. $C_3$-$C_7$-Cycloalkenyl, und bedeutet z. B Cyclopent-2-enyl oder -3-enyl oder Cyclohex-2-enyl oder -3-enyl.

Phenylniederalkyl ist Phenyl-$C_1$-$C_4$-alkyl und bedeutet z. B. Benzyl oder 1- oder 2-Phenethyl, während Phenylniederalkenyl bzw. Phenylniederalkinyl Phenyl-$C_3$-$C_5$-alkenyl bzw. -alkinyl bedeuten, z. B. 3-Phenylallyl oder 3-Phenylpropargyl.

Cycloalk-1,1-ylen ist Cycloniederalk-1,1-ylen, d. h. $C_3$-$C_7$-Cycloalk-1,1-ylen, d. h. Cycloprop-1,1-ylen, Cyclobut-1,1-ylen, Cyclopent-1,1-ylen, Cyclohex-1,1-ylen oder Cyclohept-1,1-ylen.

Niederalkylen (das definitionsgemäss nicht mit dem vorstehend definierten geradkettigen Alkylen X identisch zu sein braucht) bedeutet $C_2$-$C_7$-Alkylen, ist geradkettig oder verzweibt und bedeutet insbesondere Eth-1,2-ylen, Prop-1,3-ylen, But-1,4-ylen, Pent-1,5-ylen, Prop-1,2-ylen, 2-Methylprop- 1,3-ylen oder 2,2-Dimethylprop- 1,3-ylen. Bevorzubt ist $C_2$-$C_5$-Alkylen.

Niederalkylenoxyniederalkylen bedeutet $C_2$-$C_4$-Alkylenoxy-$C_2$-$C_4$-alkylen, z. B. Ethylenoxyethylen.

Niederalkanoyl bedeutet $C_1$-$C_7$-Alkanoyl und ist z.B. Formyl, Acetyl, Propionyl, Butyryl, Isobutyryl oder Pivaloyl. Bevorzubt ist $C_2$-$C_5$-Alkanoyl.

Halogenniederalkansulfonyl ist Halogen-$C_1$-$C_7$-alkansulfonyl, wie Trifluormethansulfonyl.

Diniederalkoxymethyl ist insbesondere Di-$C_1$-$C_4$-alkoxymethyl, z. B. Dimethoxymethyl oder Diethoxymethyl.

1,3-Dioxacycloalk-2-yl ist 1,3-Dioxa-$C_4$-$C_7$-cycloalk-2-yl, z. B. 1,3-Dioxacyclopent-2-yl oder 1,3-Dioxacyclohex-2-yl.

Niederalkoxy ist $C_1$-$C_7$-Alkoxy, d. h. Methoxy, Ethoxy, n-Propyloxy, Isopropyloxy, n-Butyloxy, Isobutyloxy, sek.-Butyloxy, tert.-Butyloxy oder entsprechendes Pentyloxy, Hexyloxy oder Heptyloxy. Bevorzugt ist $C_1$-$C_4$-Alkoxy.

Niederalkenyloxy bedeutet $C_3$-$C_7$-Alkenyloxy und ist z.B. Allyloxy, But-2-en-1-yloxy oder But-3-en-1-yloxy. Bevorzugt ist $C_3$-$C_5$-Alkenyloxy.

Ausgedehnte pharmakologische Untersuchungen haben ergeben, dass die Verbindungen I und ihre pharmazeutisch verwendbaren Salze z. B. ausgeprägte Angiotensin-II-antagonisierende Eigenschaften aufweisen.

Bekanntlich hat Angiotensin-II starke vasokonstriktorische Eigenschaften und stimuliert ausserdem die Aldosteronsekretion und bewirkt somit eine deutliche Natrium/Wasser-Retention. Die Folge der Angiotensin-II-Aktivität manifestiert sich unter anderem in einer Erhöhung des Blutdrucks.

Die Bedeutung von Angiotensin-II-Antagonisten besteht darin, durch kompetitive Hemmung der Bindung von Angiotensin-II an die Rezeptoren die durch Angiotensin-II bewirkten vasokonstriktorischen und die Aldosteronsekretion-stimulierenden Effekte zu unterdrücken.

Die Angiotensin-II-antagonisierenden Eigenschaften der Verbindungen I und ihrer pharmazeutisch verwendbaren Salze können im Angiotensin-II-Bindungstest erfasst werden. Dabei werden glatte Muskelzellen der Ratte aus homogenisierter Rattenaorta verwendet. Das feste Zentrifugat wird in 50 mM Tris-Puffer (pH 7.4) unter Einsatz von Peptidaseinhibitoren suspendiert. Die Proben werden 60 Minuten bei 25°C mit $^{125}$I-Angiotensin-II (0. 175 nM) und einer variierenden Konzentration an Angiotensin-II oder an Testsubstanz inkubiert.

Die Inkubation wird dann durch Zugabe von mit eiskaltem Phosphat gepuffertem Kochsalz beendet und es wird durch Whatman GF/F Filter filtriert. Die Filter werden mit einem Gamma-Zähler gezählt. Aus der Dosis-Wirkungs-Kurve werden die $IC_{50}$-Werte bestimmt. Für die Verbindungen I und ihre pharmazeutisch verwendbaren Salze werden $IC_{50}$-Werte ab etwa 10 nM ermittelt.

Zur Bestimmung der Angiotensin-II induzierten Vasokonstriktion können Untersuchungen an dem isolierten Kaninchen-Aortaring herangezogen werden. Hierzu werden von jeder Brust Aortaringe präpariert und zwischen 2 parallelen Klammern bei einer anfänglich bestehenden Spannung von 2 g fixiert. Anschliessend werden die Ringe bei 37°C in 20 ml eines Gewebebades getaucht und mit einem Gemisch aus 95 % $O_2$ und 5 % $CO_2$ begast. Die isometrischen Reaktionen werden gemessen. In 20-minütigen Intervallen werden die Ringe abwechselnd mit 10 nM Angiotensin-II (Hypertensin-CIBA) und 5 nM Noradrenalinchlorid stimuliert. Anschliessend werden die Ringe mit ausgewählten Konzentrationen der Testsubstanzen vor der Behandlung mit den Agonisten inkubiert. Die Daten werden mit einem Buxco Digitalcomputer analysiert. Die Konzentrationen, die eine 50%-ige Hemmung der Anfangskontrollwerte bewirken, werden als $IC_{50}$-Werte angegeben. Für die Verbindungen I und ihre pharmazeutisch verwendbaren Salze werden $IC_{50}$-Werte ab etwa 5 nM bestimmt.

Dass die Verbindungen I und ihre pharmazeutisch verwendbaren Salze durch Angiotensin-II induzierten Bluthochdruck reduzieren können, kann im Testmodell der normotensiven, narkotisierten Ratte verifiziert werden. Nach Kalibration der Präparationen mitjeweils 0.9 % NaCl (1 ml/kg i.v.), Noradrenalin (1 µg/kg i.v.) bzw. Angiotensin-II (0.3 µg/kg i.v.) werden steigende Dosen (3-6) der Testsubstanz durch Bolusinjektion intravenös injiziert, worauf nach jeder Dosis in 5 Minuten-Intervallen Angiotensin-II bzw. Noradrenalin appliziert wird. Der Blutdruck wird direkt in der Halsschlagader gemessen und mit einem on-line Datenerfassungssystem aufgezeichnet (Buxco). Die Spezifität des Angiotensin-II-Antagonismus wird angezeigt durch die selektive Hemmung des von Angiotensin-II, nicht aber des durch Noradrenalin hervorgerufenen Druckeffektes. In diesem Testmodell zeigen die Verbindungen I und ihre pharmazeutisch verwendbaren Salze ab einer Dosis von etwa 0.3 mg/kg i.v. einen hemmenden Effekt.

Auch im Testmodell der renalen hypertensiven Ratte kann die antihypertensive Aktivität der Verbindungen I und ihrer pharmazeutisch verwendbaren Salze manifestiert werden. Bei männlichen Ratten wird durch Verengung einer renalen Arterie gemäss der Goldblatt-Methode Bluthochdruck erzeugt. Den Ratten werden mittels einer Magensonde Dosen der Testsubstanz verabreicht. Kontrolltiere erhalten ein äquivalentes Volumen an Lösungsmittel. Blutdruck und Herzschlag werden indirekt an wachen Tieren nach der Schwanzklemm-Methode von Gerold et al. [Helv. Physiol. Acta 24 (1966), 58] vor Verabreichung der Testsubstanz bzw. des Lösungsmittels sowie während des Verlaufs der Experimente in Intervallen gemessen. Der ausgeprägte antihypertensive Effekt kann ab einer Dosis von etwa 30 mg/kg p.o. nachgewiesen werden.

Dementsprechend können die Verbindungen I und ihre pharmazeutisch verwendbaren Salze z.B. als Wirkstoffe in Antihypertensiva verwendet werden, welche z.B. zur Behandlung von Bluthochdruck sowie von Herzinsuffiiienz eingesetzt werden. Ein Erfindungsgegenstand ist somit die Verwendung der Verbindungen I und ihrer pharmazeutisch verwendbaren Salze zur Herstellung von entsprechenden Arzneimitteln und zur therapeutischen Behandlung von Bluthochdruck sowie von Herzinsuffizienz. Bei der Herstellung der Arzneimittel ist auch die gewerbsmässige Herrichtung der Wirksubstanzen eingeschlossen.

Bevorzugt sind Verbindungen der Formel I, worin $R_1$ Niederalkyl, Niederalkenyl, Niederalkinyl, Halogen in höherer als der β-Stellung zu dem in der Formel I gezeigten Stickstoffatom tragendes Halogen-niederalkyl, -niederalkenyl oder -niederalkinyl, Hydroxy in höherer als der β-Stellung zu dem in der Formel I gezeigten Stickstoffatom tragendes Hydroxy-niederalkyl, -niederalkenyl oder -niederalkinyl, Cycloniederalkyl, Cycloniederalkenyl, Phenylniederalkyl oder Phenyl-niederalkenyl oder -niederalkinyl ist; $R_2$ 1H-Tetrazol-5-yl, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, Amino, durch Niederalkyl, Niederalkenyl, Niederalkinyl oder Phenyl-niederalkyl, -niederalkenyl oder -niederalkinyl mono- oder unabhängig voneinander disubstituiertes oder durch Niederalkylen oder Niederalkylenoxyniederalkylen disubstituiertes Amino, durch Niederalkanoyl oder Benzoyl monosubstituiertes Amino, Formyl, Diniederalkoxymethyl, 1,3-Dioxacycloalk-2-yl, Hydroxy, Niederalkoxy, Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkanoyl, Aminosulfonyl, Aminosulfonyl, in welchem die Aminogruppe durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, $PO_2H_2$, $PO_3H_2$ oder Sulfonylamino, in welchem das Schwefelatom der Sulfonylaminogruppe durch Niederalkyl, Niederalkenyl, Niederalkinyl, Halogen-niederalkyl, -niederalkenyl oder -niederalkinyl, Phenyl-niederalkyl, -niederalkenyl oder -niederalkinyl oder Phenyl mono-

substituiert ist, bedeutet, $R_3$ Carboxy, 1H-Tetrazol-5-yl, $SO_3H$, $PO_2H_2$, $PO_3H_2$ oder Halogenniederalkansulfonylamino ist; X geradkettiges Niederalkylen, geradkettiges Niederalkylen, in welchem eine oder unabhängig voneinander zwei Methylengruppe(n) durch Cycloniederalk-1,1-ylen ersetzt ist (sind), geradkettiges Niederalkylen, das ein- oder unabhängig voneinander zwei-, drei- oder vierfach durch Niederalkyl, Niederalkenyl oder Niederalkinyl substituiert ist, oder geradkettiges Niederalkylen, das ein- oder unabhängig voneinander zwei-, drei- oder vierfach durch Niederalkyl, Niederalkenyl oder Niederalkinyl substituiert ist und in welchem eine oder unabhängig voneinander zwei Methylengruppe(n) der in der entsprechenden unsubstituierten geradkettigen Niederalkylengruppe vorhandenen Methylengruppen durch Cycloniederalk-1,1-ylen ersetzt ist (sind), darstellt oder für das Strukturelement der Formel $-X_1-Ph-X_2-$ steht, wobei $X_1$ und $X_2$ unabhängig voneinander für eine Bindung oder für $C_1-C_7$-Alklyen stehen und Ph Phenylen bedeutet; wobei die Ringe A und B unabhängig voneinander unsubstituiert oder durch Halogen, Hydroxy, Niederalkoxy, Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkyl, Niederalkenyl, Niederalkinyl, Halogen-niederalkyl, -niederalkenyl oder -niederalkinyl, Hydroxy-niederalkyl, -niederalkenyl oder -niederalkinyl, gegebenenfalls durch Halogen oder Hydroxy substituiertes Niederalkoxy-niederalkyl, -niederalkenyl oder -niederalkinyl oder gegebenenfalls durch Halogen oder Hydroxy substituiertes Niederalkenyloxy-niederalkyl, -niederalkenyl oder -niederalkinyl substituiert sind; und entweder p und q beide für 1 stehen oder p 0 ist und q 0 oder 1 darstellt; wobei Phenyl in Phenylresten per se und in Phenylen-, Phenylniederalkyl-, Phenylniederalkenyl-, Phenylniederalkinyl-, Benzoyl-, Phenoxy- und Benzyloxy-Gruppen jeweils unsubstituiertes oder ein- oder unabhängig voneinander mehrfach durch (einen) Substituenten ausgewählt aus der Gruppe, die aus Halogen, Hydroxy, Niederalkoxy, Niederalkyl und Trifluormethyl besteht, substituiertes Phenyl bedeutet; in freier Form oder in Salzform.

Besonders bevorzugt sind Verbindungen der Formel I, worin $R_1$ Niederalkyl, Niederalkenyl oder Cycloniederalkyl ist; $R_2$ 1H-Tetrazol-5-yl, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, Amino, durch Niederalkyl, Niederalkenyl, Niederalkinyl oder Phenyl-niederalkyl, -niederalkenyl oder -niederalkinyl mono- oder unabhängig voneinander disubstituiertes oder durch Niederalkylen oder Niederalkylenoxyniederalkylen disubstituiertes Amino, durch Niederalkanoyl oder Benzoyl monosubstituiertes Amino, Formyl, Diniederalkoxymethyl, 1,3-Dioxacycloalk-2-yl, Hydroxy, Niederalkoxy, Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkanoyl, Aminosulfonyl, Aminosulfonyl, in welchem die Aminogruppe durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, $PO_2H_2$, $PO_3H_2$ oder Sulfonylamino, in welchem das Schwefelatom der Sulfonylaminogruppe durch Niederalkyl, Niederalkenyl, Niederalkinyl, Halogen-niederalkyl, -niederalkenyl oder -niederalkinyl, Phenyl-niederalkyl, -niederalkenyl oder -niederalkinyl oder Phenyl monosubstituiert ist, bedeutet; $R_3$ Carboxy, 1H-Tetrazol-5-yl oder Halogenniederalkansulfonylamino ist; X geradkettiges Niederalkylen, geradkettiges Niederalkylen, in welchem eine Methylengruppe durch Cycloniederalk-1,1-ylen ersetzt ist, geradkettiges Niederalkylen, das ein- oder unabhängig voneinander zwei-, drei- oder vierfach durch Niederalkyl, Niederalkenyl oder Niederalkinyl substituiert ist, oder geradkettiges Niederalkylen, das ein- oder unabhängig voneinander zweifach durch Niederalkyl, Niederalkenyl oder Niederalkinyl substituiert ist und in welchem eine Methylengruppe der in der entsprechenden unsubstituierten geradkettigen Niederalkylengruppe vorhandenen Methylengruppen durch Cycloniederalk-1,1-ylen ersetzt ist, darstellt; oder X für das Strukturelement der Formel $-X_1-Ph-X_2-$ steht, wobei $X_1$ und $X_2$ unabhängig voneinander für eine Bindung oder für $C_1-C_7$-Alkylen stehen und Ph Phenylen bedeutet; die Ringe A und B unabhängig voneinander unsubstituiert oder durch Halogen, Hydroxy, Niederalkoxy, $S(O)_m$-R, wobei m 0, 1 oder 2 ist und R Wasserstoff oder Niederalkyl ist, Niederalkyl, Halogen-niederalkyl, Hydroxy-niederalkyl, gegebenenfalls durch Halogen oder Hydroxy substituiertes Niederalkoxy-niederalkyl oder gegebenenfalls durch Halogen oder Hydroxy substituiertes Niederalkenyloxy-niederalkyl substituiert sind; und entweder p und q beide für 1 stehen oder p 0 ist und q 0 oder 1 darstellt; wobei Phenyl in Phenylresten per se und in Phenylen-, Phenylniederalkyl-, Phenylniederalkenyl-, Phenylniederalkinyl-, Benzoyl-, Phenoxy- und Benzyloxy-Gruppen jeweils unsubstituiertes oder ein- oder unabhängig voneinander zweifach durch (einen) Substituenten ausgewählt aus der Gruppe, die aus Halogen, Hydroxy, Niederalkoxy, Niederalkyl und Trifluormethyl besteht, substituiertes Phenyl bedeutet; in freier Form oder in Salzform.

Ganz besonders bevorzugt sind Verbindungen der Formel I, worin $R_1$ Niederalkyl oder Niederalkenyl ist; $R_1$ 1H-Tetrazol-5-yl, Carboxy, Carboxy, welches durch einen Alkohol verestert ist, dessen alkoholische Hydroxyfunktion an Niederalkyl oder Niederalkoxyniederalkyl gebunden ist, Carbamoyl, dessen Aminogruppe unsubstituiert oder durch Niederalkyl oder Phenyl-niederalkyl mono- oder unabhängig voneinander disubstituiert oder durch Niederalkylen oder Niederalkylenoxyniederalkylen disubstituiert ist, Amino, durch Niederalkyl oder Phenyl-niederalkyl mono- oder unabhängig voneinander disubstituiertes oder durch Niederalkylen oder Niederalkylenoxyniederalkylen disubstituiertes Amino, durch Niederalkanoyl monosubstituiertes Amino, Hydroxy, Niederalkoxy, Phenoxy, Benzyloxy, $S(O)_m$-R, wobei m 0, 1 oder 2 ist und R Wasserstoff oder Niederalkyl ist, Niederalkanoyl, Aminosulfonyl, $PO_2H_2$, $PO_3H_2$ oder Sulfonylamino, in welchem das Schwefelatom der Sulfonylaminogruppe durch Niederalkyl, Halogenniederalkyl, Phenylniederalkyl oder Phenyl monosubstituiert ist, bedeutet; $R_3$ Carboxy oder 1H-Tetrazol-5-yl ist; X geradkettiges Niederalkylen, geradkettiges Niederalkylen, in welchem eine Methylengruppe durch Cycloniederalk-1,1-ylen ersetzt ist, oder geradkettiges Niederalkylen, das ein- oder unabhängig voneinander zweifach durch Niederalkyl substituiert ist, darstellt, oder X für das Strukturelement der Formel $-X_1$-Ph-$X_2$- steht, wobei $X_1$ und $X_2$ unabhängig voneinander für eine Bindung oder für $C_1$-$C_7$-Alkylen stehen und Ph Phenylen bedeutet; die Ringe A und B unabhängig voneinander unsubstituiert oder durch Halogen, Hydroxy, Niederalkoxy, $S(O)_m$-R, worin m 0, 1 oder 2 ist und R Wasserstoff oder Niederalkyl ist, oder Niederalkyl substituiert sind; p 0 ist, q 0 oder 1 darstellt; und der Ring B in 4-Position des Ringes A gebunden ist; wobei Phenyl in Phenylen-, Phenylniederalkyl-, Phenoxy- und Benzyloxy-Gruppen jeweils unsubstituiertes oder einfach durch einen Substituenten ausgewählt aus der Gruppe, die aus Halogen, Niederalkoxy, Niederalkyl und Trifluormethyl besteht, substituiertes Phenyl bedeutet; in freier Form oder in Salzform.

Insbesondere bevorzugt sind Verbindungen der Formel I, worin die Ringe A und B unsubstituiert sind; p 0 ist; q 1 darstellt; der Ring B in 4-Position des Ringes A gebunden ist; und $R_3$ in einer 2-Position des Ringes B gebunden ist; in freier Form oder in Salzform.

Insbesondere bevorzugt sind Verbindungen der Formel I, worin $R_1$ $C_1$-$C_4$-Alkyl, wie Propyl oder Butyl, oder $C_3$-$C_5$-Alkenyl, wie Allyl, ist; $R_2$ 1H-Tetrazol-5-yl, Carboxy, $C_1$-$C_4$-Alkoxycarbonyl, wie Methoxy- oder Ethoxycarbonyl, Carbamoyl, N-$C_1$-$C_4$-Alkylcarbamoyl, wie N-Methylcarbamoyl, N,N-Di-$C_1$-$C_4$-alkylcarbamoyl, wie N,N-Dimethylcarbamoyl, $C_1$-$C_4$-Alkanoyl, wie Acetyl, Aminosulfonyl oder Sulfonylamino, in welchem das Schwefelatom der Sulfonylaminogruppe durch $C_1$-$C_4$-Alkyl, wie Methyl, monosubstituiert ist, bedeutet; $R_3$ Carboxy oder 1H-Tetrazol-5-yl ist; X geradkettiges $C_1$-$C_7$-Alkylen, wie Eth- 1,2-ylen, Prop- 1,3-ylen, But- 1,4-ylen oder Pent- 1,5-ylen, in welchem eine Methylengruppe durch $C_3$-$C_7$-Cycloalk-1,1-ylen, wie Cyclopent-1,1-ylen oder Cyclohex-1,1-ylen, ersetzt ist, oder geradkettiges $C_1$-$C_7$-Alkylen, wie Methylen, Eth- 1,2-ylen, Prop- 1,3-ylen oder But- 1,4-ylen, das ein- oder unabhängig voneinander zweifach durch $C_1$-$C_4$-Alkyl, wie Methyl oder Ethyl, substituiert ist, darstellt; die Ringe A und B unsubstituiert sind; p 0 ist; q 1 darstellt; der Ring B in 4-Position des Ringes A gebunden ist; und $R_3$ in einer 2-Position des Ringes B gebunden ist; in freier Form oder in Salzform.

Insbesondere bevorzugt sind Verbindungen der Formel I, worin $R_1$ $C_1$-$C_4$-Alkyl, wie Propyl oder Butyl, oder $C_3$-$C_5$-Alkenyl, wie Allyl, ist; $R_2$ 1H-Tetrazol-5-yl, Carboxy, $C_1$-$C_4$-Alkoxycarbonyl, wie Methoxy- oder Ethoxycarbonyl, Carbamoyl, N-$C_1$-$C_4$-Alkylcarbamoyl, wie N-Methylcarbamoyl, N,N-Di-$C_1$-$C_4$-alkylcarbamoyl, wie N,N-Dimethylcarbamoyl, $C_1$-$C_4$-Alkanoyl, wie Acetyl, Aminosulfonyl oder Sulfonylamino, in welchem das Schwefelatom der Sulfonylaminogruppe durch $C_1$-$C_4$-Alkyl, wie Methyl, monosubstituiert ist, bedeutet; $R_3$ Carboxy oder 1H-Tetrazol-5-yl ist; X für das Strukturelement der Formel $-X_1$-Ph-$X_2$- steht, wobei $X_1$ und $X_2$ unabhängig voneinander für eine Bindung oder für geradkettiges $C_1$-$C_3$-Alkylen, insbesondere Methylen, stehen und Ph Phenylen, wie 1,2- oder 1,3-Phenylen, bedeutet; die Ringe A und B unsubstituiert sind; p 0 ist; q 1 darstellt; der Ring B in 4-Position des Ringes A gebunden ist; und $R_3$ in einer 2-Position des Ringes B gebunden ist; in freier Form oder in Salzform.

In ganz besonderer Weise bevorzugt sind Verbindungen der Formel I, worin $R_1$ $C_1$-$C_4$-Alkyl, wie Propyl oder Butyl, ist; $R_2$ Carboxy, $C_1$-$C_4$-Alkoxycarbonyl, wie Methoxycarbonyl, oder Carbamoyl bedeutet; $R_3$ 1H-Tetrazol-5-yl ist; X geradkettiges $C_1$-$C_7$-Alkylen, wie Eth- 1,2-ylen, Prop- 1,3-ylen, But- 1,4-ylen oder Pent- 1,5-ylen, in welchem eine Methylengruppe durch $C_3$-$C_7$-Cycloalk-1,1-ylen, wie Cyclopent-1,1-ylen, ersetzt ist, oder geradkettiges $C_1$-$C_7$-Alkylen, wie Methylen, Eth-1,2-ylen, Prop-1,3-ylen oder But-1,4-ylen, das ein- oder unabhängig voneinander zweifach durch $C_1$-$C_4$-Alkyl, wie Methyl oder Ethyl, substituiert ist, darstellt; die Ringe A und B unsubstituiert sind; p 0 ist; q 1 darstellt; der Ring B in 4-Position des Ringes A gebunden ist; und $R_3$ in einer 2-Position des Ringes B gebunden ist; in freier Form oder in Salzform.

In erster Linie bevorzugt sind Verbindungen der Formel I, worin $R_1$ $C_1$-$C_4$-Alkyl, wie Propyl oder Butyl, ist; $R_2$ Carboxy bedeutet; $R_3$ 1H-Tetrazol-5-yl ist; X geradkettiges $C_1$-$C_7$-Alkylen, wie Eth- 1,2-ylen, Prop-1,3-ylen, But-1,4-ylen oder Pent-1,5-ylen, in welchem eine Methylengruppe durch $C_3$-$C_7$-Cycloalk-1,1-ylen, wie Cyclopent-1,1-ylen, ersetzt ist, oder geradkettiges $C_1$-$C_7$-Alkylen, wie Methylen, Eth-1,2-ylen, Prop-1,3-ylen oder But-1,4-ylen, das ein- oder unabhängig voneinander zweifach durch $C_1$-$C_4$-Alkyl, wie Methyl oder Ethyl, substituiert ist, darstellt; die Ringe A und B unsubstituiert sind; p 0 ist; q 1 darstellt; der Ring B in 4-Position des Ringes A gebunden ist; und $R_3$ in einer 2-Position des Ringes B gebunden ist; in freier Form oder in Salzform.

In erster Linie bevorzugt sind Verbindungen der Formel I, worin $R_1$ $C_3$-$C_4$-Alkyl, wie n-Propyl oder n-Butyl,

ist; $R_2$ Carboxy bedeutet; $R_3$ 1H-Tetrazol-5-yl ist; X 2-Methyl-but-1,3-ylen ist oder 1,3-Propylen bedeutet, in welchem die ω-Methylengruppe durch Cyclohex-1,1-ylen ersetzt ist; die Ringe A und B unsubstituiert sind; p 0 ist; q 1 darstellt; der Ring B in 4-Position des Ringes A gebunden ist; und $R_3$ in einer 2-Position des Ringes B gebunden ist; in freier Form oder in Salzform.

Namentlich bevorzugt sind die in den Beispielen genannten Verbindungen der Formel I, in freier Form oder in Salzform.

Ein weiterer Gegenstand der Erfindung ist ein Verfahren zur Herstellung der Verbindungen I und ihrer Salze, z.B. dadurch gekennzeichnet, dass man

a) in einer Verbindung der Formel

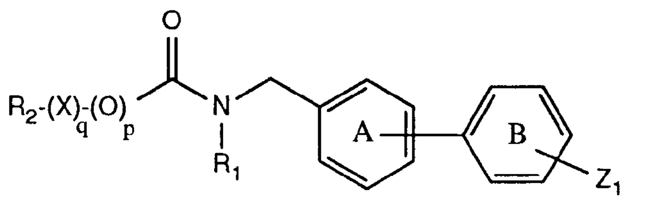

(II),

worin $Z_1$ einen in $R_3$ überführbaren Rest darstellt, oder in einem Salz davon $Z_1$ in $R_3$ überführt oder

b) eine Verbindung der Formel

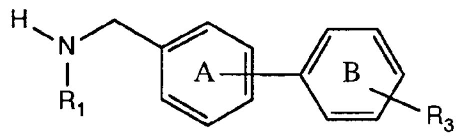

(III)

oder ein Salz davon mit einer Verbindung der Formel

$$R_2'-(X)_q-(O)_p-C(=O)-Z_2 \quad (IV)$$

worin entweder $C(=O)-Z_2$ eine Carboxygruppe oder ein reaktionsfähiges Derivat davon darstellt und $R_2'$ ein Rest $R_2$ ist oder worin $R_2'$ und $C(=O)-Z_2$ gemeinsam die Gruppe -C(=O)-O-C(=O)- bilden, oder einem Salz davon umsetzt und jeweils, wenn erwünscht, eine verfahrensgemäss oder auf andere Weise erhältliche verbindung I, in freier Form oder in Salzform, in eine andere verbindung I überführt, ein verfahrensgemäss erhältliches Gemisch von Isomeren auftrennt und das gewünschte Isomere isoliert und/oder eine verfahrensgemäss erhältliche freie verbindung I in ein Salz oder ein verfahrensgemäss erhältliches Salz einer verbindung I in die freie verbindung I oder in ein anderes Salz überführt.

Salze von Ausgangsmaterialien, die mindestens ein basisches Zentrum aufweisen, sind entsprechende Säureadditionssalze, während Salze von Ausgangsstoffen, die mindestens eine acide Gruppe aufweisen, Salze mit Basen sind, jeweils wie in Zusammenhang mit entsprechenden Salzen von Verbindungen I vorstehend aufgeführt.

Die vor- und nachstehend in den varianten beschriebenen Umsetzungen werden in an sich bekannter Weise durchgeführt, z.B. in Ab- oder üblicherweise in Anwesenheit eines geeigneten Lösungs- oder verdünnungsmittels oder eines Gemisches derselben, wobei man je nach Bedarf unter Kühlen, bei Raumtemperatur oder unter Erwärmen, z.B. in einem Temperaturbereich von etwa -80°C bis zur Siedetemperatur des Reaktionsmediums, vorzugsweise von etwa - 10° bis etwa +200°C, und, falls erforderlich, in einem geschlossenen Gefäss, unter Druck, in einer Inertgasatmosphäre und/oder unter wasserfreien Bedingungen arbeitet. Einzelheiten zu entsprechenden verfahrensweisen und Reaktionsbedingungen können insbesondere auch den Beispielen entnommen werden.

Variante a):

In die Variable $R_3$ überführbare Reste $Z_1$ sind beispielsweise Cyano, Mercapto, Halogen, die Gruppe -$N_1^+ A^-$, in der $A^-$ ein von einer Säure abgeleitetes Anion bedeutet, Amino, funktionelle Derivate von COOH, $SO_3H$, $PO_3H_2$ und $PO_2H_2$ und geschütztes 1H-Tetrazol-5-yl.

In 1H-Tetrazol-5-yl $R_3$ überführbare Reste $Z_1$ sind beispielsweise Cyano und geschütztes 1H-Tetrazol-5-yl.

Zur Herstellung von Verbindungen I, worin $R_3$ 1H-Tetrazol-5-yl bedeutet, geht man beispielsweise von Ausgangsmaterial II aus, worin $Z_1$ Cyano bedeutet, und setzt dieses, z. B. in einem inerten Lösungsmittel, wie in einem aromatischen oder araliphatischen Kohlenwasserstoff, z. B. in Toluol oder einem Xylol, vorzugsweise

unter Erwärmen, mit einem Azid, z. B. mit $HN_3$ oder insbesondere einem Salz, wie Alkalimetallsalz, davon oder mit einem Organozinnazid, wie Triniederalkyl- oder Triaryl-zinnazid, um. Bevorzugte Azide sind beispielsweise Natrium- und Kaliumazid sowie Tri-$C_1$-$C_4$-alkyl-, z.B. Trimethyl- oder Tributyl-zinnazid, und Triphenylzinnazid.

Als Schutzgruppen von geschütztem 1H-Tetrazol-5-yl kommen die üblicherweise in der Tetrazolchemie verwendeten Schutzgruppen in Frage, insbesondere Triphenylmethyl, gegebenenfalls, z.B. durch Nitro, substituiertes Benzyl, wie 4-Nitrobenzyl, Niederalkoxymethyl, wie Methoxy- oder Ethoxymethyl, Niederalkylthiomethyl, wie Methylthiomethyl, sowie 2-Cyanoethyl, ferner Niederalkoxyniederalkoxymethyl, wie 2-Methoxyethoxymethyl, Benzyloxymethyl sowie Phenacyl. Die Abspaltung der Schutzgruppen erfolgt in Anlehnung an bekannte Methoden. So wird z.B. Triphenylmethyl üblicherweise durch Hydrolyse, insbesondere in Gebenwart einer Säure, z. B. in Gegenwart von Halogenwasserstoff, vorteilhaft in einem inerten Lösungsmittel, wie in einem Halogenalkan oder einem Ether, z. B. in Dichlormethan oder Dioxan, und unter Erwärmen, oder durch Hydrogenolyse in Gegenwart eines Hydrierunbskatalysators, 4-Nitrobenzyl z.B. durch Hydrogenolyse in Gegenwart eines Hydrierungskatalysators, Methoxy- oder Ethoxy-methyl z.B. durch Behandeln mit einem Triniederalkyl-, wie Triethyl- oder Tributyl-zinn-bromid, Methylthiomethyl z. B. durch Behandeln mit Trifluoressigsäure, 2-Cyanoethyl z.B. durch Hydrolyse, beispielsweise mit Natronlauge, 2-Methoxyethoxymethyl z.B. durch Hydrolyse, z.B. mit Salzsäure, und Benzyloxymethyl und Phenacyl z.B. durch Hydrogenolyse in Gegenwart eines Hydrierungskatalysators abgespalten.

Ein in $SO_3H$ $R_3$ überführbarer Rest $Z_1$ ist beispielsweise die Mercaptogruppe. Eine solche Gruppe aufweisende Ausgangsverbindungen II werden beispielsweise durch an sich bekannte Oxidationsverfahren zu Verbindungen I oxidiert, worin $R_3$ $SO_3H$ ist. Als Oxidationsmittel kommen beispielsweise anorganische Persäuren, wie Persäuren von Mineralsäuren, z. B. Periodsäure oder Perschwefelsäure, organische Persäuren, wie Percarbon- oder Persulfon-säuren, z.B. Perameisen-, Peressig-, Trifluorperessig- oder Perbenzoe-säure oder p-Toluolpersulfonsäure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolfram-oxid, zu nennen sind. Die Oxidation wird unter milden Bedingungen, z.B. bei Temperaturen von etwa -50° bis etwa + 100°C, durchgeführt.

Unter einer in $PO_3H_2$ $R_3$ überführbaren Gruppe ist beispielsweise eine Gruppe $-N_2^+ A^-$ zu verstehen, wobei $A^-$ für ein Anion einer Säure, wie Mineralsäure, steht. Entsprechende Diazoniumverbindungen werden beispielsweise in an sich bekannter Weise mit einem P(III)-Halogenid, wie $PCl_3$ oder $PBr_3$, umgesetzt und hydrolytisch aufbearbeitet, wobei solche Verbindungen I erhältlich sind, worin $R_3$ $PO_3H_2$ ist.

Verbindungen I, worin $R_3$ $PO_2H_2$ ist, erhält man z. B. durch in üblicher Weise erfolgende Umwandlung von $Z_1$ in einer Verbindung II, worin $Z_1$ ein funktionelles Derivat von $PO_2H_2$ ist, in $PO_2H_2$.

Als in Halobenalkansulfonylamino $R_3$ überführbarer Rest $Z_1$ kommt beispielsweise Amino in Frabe. Zur Herstellung von Verbindunben 1, worin $R_3$ Halobenalkansulfonylamino bedeutet, setzt man beispielsweise entsprechende Aniline mit einer üblicherweise reaktionsfähig veresterten Halogenalkansulfonsäure um, wobei gegebenenfalls in Gegenwart einer Base gearbeitet wird. Bevorzugt als reaktionsfähig veresterte Halobenakansulfonsäuren sind die entsprechenden Halogenide, wie Chloride, z. B. Trifluormethansulfonylchlorid, und die entsprechenden Anhydride, z. B. Trifluormethansulfonsäureanhydrid.

Ein in COOH $R_3$ überführbarer Rest $Z_1$ steht beispielsweise für funktionell abgewandeltes Carboxy, wie Cyano, verestertes oder amidiertes Carboxy, Hydroxymethyl oder Formyl.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, n-Propanol, Isopropanol, n-Butanol, sek.-Butanol oder tert.-Butanol, während als cycloaliphatischer Alkohol beispielsweise ein 3- bis 8-gliedriges Cycloalkanol, wie Cyclo-pentanol, -hexanol oder -heptanol, in Frage kommt. Ein aromatischer Alkohol ist beispielsweise ein Phenol oder ein heterocyclischer Alkohol, welche jeweils substituiert sein können, insbesondere Hydroxypyridin, z.B. 2-, 3- oder 4-Hydroxypyridin.

Amidiertes Carboxy ist beispielsweise Carbamoyl, durch Hydroxy, Amino oder gegebenenfalls substituiertes Phenyl monosubstituiertes, durch Niederalkyl mono- oder disubstituiertes oder durch 4- bis 7-gliedriges Alkylen oder 3-Aza-, 3-Niederalkylaza-, 3-Oxa- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- und N,N-Dipropyl-carbamoyl, Pyrrolidino- und Piperidinocarbonyl, Morpholino-, Piperazino-, 4-Methylpiperazino- und Thiomorpholino-carbonyl, Anilinocarbonyl und durch Niederalkyl, Niederalkoxy und/oder Halogen substituiertes Anilinocarbonyl zu nennen.

Bevorzugtes funktionell abgewandeltes Carboxy ist Niederalkoxycarbonyl, wie Methoxyoder Ethoxycarbonyl, und Cyano.

Verbindungen I, worin $R_3$ Carboxy ist, können beispielsweise ausgehend von Verbindungen II, worin $Z_1$ Cyano oder verestertes oder amidiertes Carboxy bedeutet, durch Hydrolyse, insbesondere in Gegenwart einer Base, oder, ausgehend von Verbindungen II, worin $Z_1$ Hydroxymethyl oder Formyl bedeutet, durch Oxidation hergestellt werden. Die Oxidation erfolgt beispielsweise in einem inerten Lösungsmittel, wie in einer Niederalkancarbonsäure, z.B. Essigsäure, einem Keton, z.B. Aceton, einem Ether, z.B. Tetrahydrofuran, einem heterocyclischen Aromaten, z.B. Pyridin, oder in Wasser, oder in einem Gemisch davon, erforderlichenfalls unter Kühlen oder Erwärmen, z.B. in einem Temperaturbereich von etwa 0° bis etwa +150°C. Als Oxidationsmittel kommen beispielsweise oxidierende Übergangsmetallverbindungen, insbesondere solche mit Elementen der I., VI. oder VII. Nebengruppe, in Frage. Als Beispiele seien genannt: Silberverbindungen, wie Silber-nitrat, -oxid und -picolinat, Chromverbindungen, wie Chromtrioxid und Kaliumdichromat, und Manganverbindungen, wie Kalium-, Tetrabutylammonium- und Benzyltriethylammonium-permanganat. Weitere Oxidationsmittel sind beispielsweise geeignete Verbindungen mit Elementen der IV. Hauptgruppe, wie Bleidioxid, oder Halogen-Sauerstoff-Verbindungen, wie Natriumiodat oder Kaliumperiodat.

Das Ausgangsmaterial II ist in Analogie zu bekannten Methoden zugänglich, beispielsweise indem man eine Verbindung der Formel

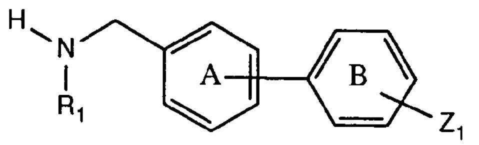

(IIa)

oder ein Salz davon mit einer Verbindung $R_2'-(X)_q-(O)_p-C(=O)-Z_2$ (IV) oder einem Salz davon umsetzt, z. B. in analoger Weise wie unter der Verfahrensvariante b) für die Umsetzung von Verbindungen III und IV erläutert.

Auf diese Weise erhältliche Verbindungen II, worin $R_2$ Carboxy ist, können gewünschtenfalls vor der Umsetzung zu Verbindungen I in an sich bekannter Weise in Verbindungen II überführt werden, worin $R_2$ verestertes Carboxy, z. B. Niederalkoxycarbonyl, ist.

Die Verbindungen IV sind bekannt oder können in an sich bekannter Weise hergestellt werden.

Die Verbindungen IIa sind in Analogie zu bekannten Methoden erhältlich, z. B. indem man eine Verbindung der Formel $R_1-NH_2$ (IIb) oder ein Salz davon in üblicher Weise mit einer Verbindung der Formel

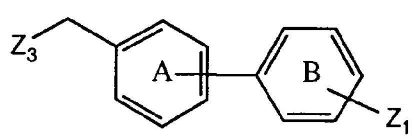

(IIc),

worin $Z_3$ für eine nucleofuge Abgangsgruppe, wie Halogen, z. B. Chlor oder Brom, Hydroxy, Niederalkoxy, z. B. Methoxy oder Ethoxy, Niederalkylthio, z. B. Methylthio, gegebenenfalls substituiertes Amino, z. B. Amino, N-Niederalkylamino oder N,N-Diniederalkylamino, oder Sulfonyloxy, z. B. gegebenenfalls halogeniertes Niederalkansulfonyloxy oder gegebenenfalls substituiertes Benzolsulfonyloxy, wie Methan-, Ethan-, Trifluormethan-, Benzol- oder p-Toluol-sulfonyloxy, steht, oder einem Salz davon umsetzt.

Die Verbindungen IIb und IIc sind bekannt (vergl. EP 253,310) oder können in an sich bekannter Weise hergestellt werden

Variante b):

Reaktionsfähige Derivate einer Carboxygruppe $C(=O)-Z_2$ sind beispielsweise von Carboxy abgeleitete aktivierte Ester, reaktionsfähige Anhydride und reaktionsfähige cyclische Amide.

Von Carboxy abgeleitete aktivierte Ester sind beispielsweise am Verknüpfungskohlenstoffatom des veresternden Restes ungesättigte Ester, z.B. Ester vom Vinylester-Typ, wie Vinylester (erhältlich z. B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode) oder 1-Niederalkoxyvinylester (erhältlich z.B. durch Behandeln der entsprechenden Säure mit einem Niederalkoxyacetylen; Ethoxyacetylen-Methode), Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode) oder N,N-disubstituierte Amidinoester (erhältlich z.B. durch Behandeln der entsprechenden Säure mit

einem N,N-disubstituierten Cyanamid; Cyanamid-Methode), oder geeignete Arylester, insbesondere durch elektronenanziehende Substituenten substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N′-Dicyclohexylcarbodiimid; Methode der aktivierten Arylester), weiterhin Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester) und insbesondere Amino- oder Amidoester (erhältlich z.B. durch Behandeln der entsprechenden Säure mit einer N-Hydroxyamino- oder N-Hydroxyamido-Verbindung oder einem aktivierten Derivat davon, z.B. mit N-Hydroxysuccinimid, N-Hydroxypiperidin, N-Hydroxyphthalimid, N-Hydroxy-5-norbornan- oder norbornan-2,3-dicarbonsäureimid, 1-Hydroxybenzotriazol, einem Benzotriazol-1-yloxy-phosphoniumsalz oder Benzotriazol-1-yluroniumsalz oder 3-Hydroxy-3,4-dihydro-1,2,3-benzotriazin-4-on, z.B. nach der Anhydrid- oder Carbodiimid-Methode; Methode der aktivierten N-Hydroxyester).

Von Carboxy abgeleitete reaktionsfähige Anhydride können vorzugsweise gemischte Anhydride sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), wie Azide (erhältlich z.B. aus einem entsprechenden Säureester über das entsprechende Hydrazid und dessen Behandlung mit salpetriger Säure; Azidmethode), wie Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy- 1,2-dihydrochinolin, z.B. 1-Ethoxycarbonyl-2-ethoxy- 1,2-dihydrochinolin; Methode der gemischten O-Alkylkohlensäureanhydride), wie Anhydride mit dihalogenierter, insbesondere dichlorierter, Phosphorsäure (erhältlich z. B. durch Behandeln der entsprechenden Säure mit Phosphoroxychlorid; Phosphoroxychloridmethode), wie Anhydride mit anderen Phosphorsäurederivaten (z.B. solchen, die man mit Phenyl-N-phenylphosphoramidochloridat erhalten kann) oder wie Anhydride mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressibsäurechlorid; Methode der gemischten Carbonsäureanhydride) oder wie Anhydride mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), können weiterhin aber auch symmetrische Anhydride (erhältlich z.B. durch Kondensation der entsprechenden Säure in Gegenwart eines Carbodiimids oder von 1-Diethylaminopropin; Methode der symmetrischen Anhyride) sein.

Von Carboxy abgeleitete reaktionsfähige cyclische Amide sind insbesondere Amide mit fünfgliedrigen Diazacyclen aromatischen Charakters, wie Amide mit Imidazolen, z.B. mit Imidazol (erhältlich z.B. durch Behandeln der entsprechenden Säure mit N,N′-Carbonyldiimidazol; Imidazol-Methode), oder Pyrazolen, z.B. mit 3,5-Dimethylpyrazol (erhältlich z.B. über das Säurehydrazid durch Behandeln mit Acetylaceton; Pyrazolid-Methode).

Die Kondensation zur Herstellung der Amidbindung wird in an sich bekannter Weise durchgeführt, beispielsweise wie in Standardwerken [z. B. in "Houben-Weyl - Methoden der organischen Chemie" (Band 15/II, 4. Auflage, Georg Thieme Verlag, Stuttgart, 1974), in "The Peptides" (Herausg.: E. Gross und J. Meienhofer, Bände 1 und 2, Academic Press, London und New York, 1979/1980) oder in "Principles of Peptide Synthesis" (Herausg.: M. Bodanszky, Springer-Verlag, Berlin, 1984)] beschrieben.

Die Kondensation kann in Gegenwart eines der üblichen Kondensationsmittel durchgeführt werden. Uebliche Kondensationsmittel sind z. B. Carbodiimide, beispielsweise Diethyl-, Dipropyl-, N-Ethyl-N′-(3-dimethylaminopropyl)- oder insbesondere Dicyclohexyl-carbodiimid, ferner geeignete Carbonylverbindungen, beispielsweise Carbonyldiimidazol, 1,2-Oxazolium verbindungen, z.B. 2-Ethyl-5-phenyl- 1,2-oxazolium-3′-sulfonat oder 2-tert-Butyl-5-methylisoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoramidochloridat, Bis(2-oxo-3-oxazolidinyl)phosphinsäurechlorid oder 1-Benzotriazolyloxy-tris(dimethylamino)phosphonium-hexafluorophosphat.

Gewünschtenfalls wird eine organische Base zugegeben, z.B. ein Triniederalkylamin mit voluminösen Resten, z.B. Ethyldiisopropylamin, oder eine heterocyclische Base, z.B. Pyridin, 4-Dimethylaminopyridin oder bevorzugt N-Methylmorpholin. Die Kondensation von Säureanhydriden mit Aminen kann z.B. in Gegenwart von anorganischen Carbonaten, z.B. Alkalimetallcarbonaten oder -hydrogencarbonaten, wie Natrium- oder Kaliumcarbonat oder -hydrogencarbonat (üblicherweise zusammen mit einem Sulfat), erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. in Formamid oder N,N-Dimethylformamid, in einem halogenierten Kohlenwasserstoff, z.B. in Dichlormethan, Tetrachlormethan oder Chlorbenzol, in einem Keton, z.B. in Aceton, in einem cyclischen Ether, z.B. in Tetrahydrofuran, in einem Ester, z.B. in Essigsäureethylester, oder in einem Nitril, z.B. in Acetonitril, oder in Mischungen davon, gegebenenfalls bei emiedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas, z.B. in einer Stickstoffatmosphäre.

Reaktionsfähige Derivate einer Carboxygruppe C(=O)-$Z_2$ können auch in situ gebildet werden.

In einer besonders bevorzugten Ausführungsform zur Herstellung von Verbindungen I, worin $R_2$ Carboxy ist, kommen vorteilhaft solche Anhydride IV zur Anwendung, in denen $R_2'$ und C(=O)-$Z_2$ gemeinsam die Gruppe -C(=O)-O-C(=O)- bilden (Methode der inneren Anhydride), z. B. entsprechende, gegebenenfalls substituierte, Bemstein- oder Glutar-säureanhydride.

Die Verbindungen III sind in Analogie zu bekannten Methoden erhältlich, z. B. indem man eine Verbindung der Formel IIb oder ein Salz davon in üblicher Weise mit einer Verbindung der Formel

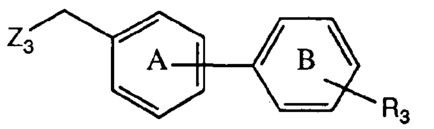

(IIIa),

worin $Z_3$ für eine nucleofuge Abgangsgruppe, wie Halogen, z. B. Chlor oder Brom, Hydroxy, Niederalkoxy, z. B. Methoxy oder Ethoxy, Niederalkylthio, z. B. Methylthio, gegebenenfalls substituiertes Amino, z. B. Amino, N-Niederalkylamino oder N,N-Diniederalkylamino, oder Sultonyloxy, z. B. gegebenenfalls halogeniertes Niederalkansulfonyloxy oder gegebenenfalls substituiertes Benzolsulfonyloxy, wie Methan-, Ethan-, Trifluormethan-, Benzol- oder p-Toluol-sulfonyloxy, steht, oder einem Salz davon umsetzt.

Die Verbindungen IIIa sind bekannt (vergl. EP 253,310) oder können in an sich bekannter Weise hergestellt werden.

Eine verfahrensgemäss oder auf andere Weise erhältliche Verbindung I kann in an sich bekannter Weise in eine andere Verbindung I überführt werden.

Beispielsweise kann eine Hydroxy aufweisende Verbindung I nach an sich bekannten Methoden verethert werden. Die Veretherung kann z.B. mit einem Alkohol, wie einem Niederalkanol, oder einem reaktionsfähigen Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide oder Methan-, Benzol- oder p-Toluol-sulfonate. Die Veretherung kann z.B. in Gegenwart einer Base, z. B. eines Alkalimetall-hydrids, -hydroxids oder -carbonats oder eines basischen Amins, erfolgen. Umgekehrt können entsprechende Ether, wie Niederalkoxyverbindungen, z.B. mittels starker Säuren, wie Mineralsäuren, z.B. Brom- oder Iod-wasserstoffsäure, die vorteilhaft in Form von Pyridiniumhalogeniden vorliegen können, oder mittels Lewissäuren, z.B. Halogeniden von Elementen der III. Hauptgruppe oder der entsprechenden Nebengruppen, gespalten werden. Diese Umsetzungen können, falls erforderlich, unter Kühlen oder Erwärmen, z.B. in einem Temperaturbereich von etwa -20° bis etwa + 100°C, in An- oder Abwesenheit eines Lösungs- oder Verdünnungsmittels, unter Inertgas und/oder unter Druck und gegebenenfalls in einem geschlossenen Gefäss durchgeführt werden.

Niederalkylthiosubstituenten kann man auf übliche Weise zu entsprechendem Niederalkan-sulfinyl oder -sulfonyl oxidieren. Als geeignetes Oxidationsmittel für die Oxidation zur Sulfoxidstufe kommen beispielsweise anorbanische Persäuren, wie Persäuren von Mineralsäuren, z.B. Periodsäure oder Perschwefelsäure, organische Persäuren, wie Percarbon- oder Persulfon-säuren, z.B. Perameisen-, Peressig-, Trifluorperessig-, Perbenzoe- oder p-Toluolpersulfon-säure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gebebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolfram-oxid, zu nennen sind. Die Oxidation wird unter milden Bedingungen, z.B. bei Temperaturen von etwa -50° bis etwa +100°C, durchgeführt. Die weitere Oxidation zur Sulfonstufe kann man mit Distickstofftetroxid als Katalysator in Gegenwart von Sauerstoff bei tiefen Temperaturen entsprechend durchführen, ebenso wie die direkte Oxidation von Niederalkylthio zu Niederalkansulfonyl. Jedoch setzt

man hierbei üblicherweise das Oxidationsmittel im Überschuss ein.

Weist eine der Variablen Amino auf, können entsprechende Verbindungen I in an sich bekannter Weise N-(ar)alkyliert werden; ebenso können Aminosulfonyl und Carbamoyl N-(ar)alkyliert werden. Die (Ar-)Alkylierung erfolgt z.B. mit einem (Aryl-)$C_1$-$C_7$-Alkyl-halogenid, z.B. -bromid oder -iodid, (Aryl-)$C_1$-$C_7$-Alkansulfonat, z.B. mit Methansulfonat oder p-Toluolsultonat, oder einem Di-$C_1$-$C_7$-alkylsulfat, z.B. Dimethylsulfat, vorzugsweise unter basischen Bedingungen, wie in Gegenwart von Natronlauge oder Kalilauge, und vorteilhaft in Gegenwart eines Phasentransfer-Katalysators, wie von Tetrabutylammoniumbromid oder Benzyltrimethylammoniumchlorid, wobei indes stärker basische Kondensationsmittel, wie Alkalimetall-amide, -hydride oder -alkoholate, z.B. Natriumamid, Natriumhydrid oder Natriumethanolat, erforderlich sein können.

In an sich bekannter Weise kann weiterhin Amino zu Acylamino acyliert werden, z. B. in analoger Weise wie unter der Verfahrensvariante b) beschrieben.

In Verbindungen I, die als Substituenten eine veresterte oder amidierte Carboxygruppe aufweisen, kann man eine solche Gruppe in üblicher Weise, z.B. mittels Hydrolyse, z.B. in Gegenwart eines basischen Mittels oder eines sauren Mittels, wie einer Mineralsäure, in die freie Carboxygruppe überführen.

In einer besonders bevorzugten Ausführungsform des Verfahrens kann man die Verfahrensvariante a) mit der Verseifung einer Carbonsäureestergruppe $R_2$ kombinieren, beispielsweise eine Verbindung II, worin $Z_1$ Cyano und $R_2$ verestertes Carboxy, insbesondere Niederalkoxycarbonyl, ist, in einem Schritt in eine Verbindung I überführen, in welcher $R_2$ Carboxy und $R_3$ 1H-Tetrazol-5-yl ist. Entsprechende vorteilhafte Verfahrensweisen und Reaktionsbedingungen können den Beispielen entnommen werden.

Ferner kann man in Verbindungen I, die als Substituenten eine Carboxygruppe aufweisen (insbesondere, sofern $R_3$ von Carboxy verschieden ist), diese, z.B. durch Behandeln mit einem Alkohol, wie einem Niederalkanol, in Gegenwart eines geeigneten Veresterungsmittels, wie eines sauren Reagens, z.B. einer anorganischen oder organischen Säure oder einer Lewissäure, z. B. von Zinkchlorid, oder eines wasserbindenden Kondensationsmittels, z.B. eines Carbodiimids, wie von N,N′-Dicyclohexylcarbodiimid, oder durch Behandeln mit einem Diazoreagens, wie mit einem Diazoniederalkan, z.B. Diazomethan, in eine veresterte Carboxygruppe überführen. Diese kann man auch erhalten, wenn man Verbindungen I, worin die Carboxygruppe in freier Form oder in Salz-, wie Ammoniumoder Metall-, z.B. Alkalimetall-, wie Natrium- oder Kalium-salzform vorliegt, mit einem $C_1$-$C_7$-Alkylhalogenid, z.B. Methyl- oder Ethyl-bromid oder -iodid, oder einem organischen Sulfonsäureester, wie einem entsprechenden $C_1$-$C_7$-Alkylester, z.B. Methansulfonsäure- oder p-Toluolsulfonsäure-methylester oder -ethylester, behandelt.

Verbindungen I, die als Substituenten eine veresterte Carboxygruppe aufweisen, kann man durch Umesterung, z.B. durch Behandeln mit einem Alkohol, üblicherwiese mit einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetall-$C_1$-$C_7$-alkanoats, -$C_1$-$C_7$-alkanolats oder -cyanids, wie von Natrium-acetat, -methanolat, -ethanolat, -tert.-butanolat oder -cyanid, oder eines geeigneten sauren Mittels, gegebenenfalls unter Entfernung des entstehenden Alkohols, z.B. durch Destillation, in andere Esterverbindunben I umwandeln. Man kann auch von entsprechenden, sobenannten aktivierten Estern I ausbehen, die als Substituenten eine aktivierte veresterte Carboxygruppe aufweisen (siehe unten), und diese durch Behandeln mit einem $C_1$-$C_7$-Alkanol in einen anderen Ester umwandeln.

Man kann in Verbindungen I, die als Substituenten die Carboxybruppe enthalten, diese auch zuerst in ein reaktionsfähiges Derivat, wie ein Anhydrid (auch ein gemischtes Anhydrid), ein Säure-halogenid, z.B. -chlorid (z.B. durch Behandeln mit einem Thionyl-halogenid, z.B. -chlorid), ein Anhydrid mit einem Ameisensäure-ester, z.B. -$C_1$-$C_7$-alkylester (z.B. durch Behandeln eines Salzes, wie eines Ammonium- oder Alkalimetallsalzes, mit einem Halogen-, wie Chlor-ameisensäureester, wie $C_1$-$C_7$-Alkylester), oder einen aktivierten Ester, wie Cyanmethyl-, Nitrophenyl-, z.B. 4-Nitro-phenyl-, oder Polyhalogenphenyl-, z.B. Pentachlorphenyl-ester (z.B. durch Behandeln mit einer entsprechenden Hydroxyverbindung in Gegenwart eines geeigneten Kondensationsmittels, wie von N,N′-Dicyclohexylcarbodiimid) überführen, und ein solches reaktionsfähiges Derivat dann mit einem Amin umsetzen und so zu Amidverbindungen I gelangen, die als Substituenten eine amidierte Carboxygruppe aufweisen. Dabei kann man diese direkt oder über Zwischenverbindungen erhalten; so kann man z.B. einen aktivierten Ester, wie einen 4-Nitrophenylester, einer Verbindung I mit einer Carboxygruppe zuerst mit einem 1-unsubstituierten Imidazol umsetzen und die so entstandene 1 -Imidazolylcarbonylverbindung mit einem Amin in Reaktion bringen. Man kann aber auch andere, nicht-aktivierte Ester, wie $C_1$-$C_7$-Alkylester, von Verbindungen I mit Aminen zur Reaktion bringen.

Weist ein aromatischer Ring als Substituenten ein Wasserstoffatom auf, so kann dieses mit Hilfe eines Halogenierungsmittels in üblicher Weise durch ein Halogenatom ersetzt werden, z.B. mit Brom, Hypobromsäure, einem Acylhypobromit oder einer anderen organischen Bromverbindung, z.B. N-Bromsuccinimid, N-Bromacetamid, N-Bromphthalimid, Pyridiniumperbromid, Dioxandibromid, 1,3-Dibrom-5,5-dimethylhydantoin oder 2,4,4,6-Tetrabrom-2,5-cyclohexandien-1-on, durch Brom oder mit elementarem Chlor, z.B. in einem halogenierten Kohlenwasserstoff, wie Chloroform, und unter Kühlen, z.B bis auf etwa - 10°C, durch Chlor.

Formyl kann in üblicher Weise, z. B. durch Umsetzung mit einem Niederalkanol oder einem Niederalkandiol, in acetalisiertes Formyl überführt werden.

Enthalten die Verbindungen I ungesättigte Reste, wie Niederalkenyl- oder Niederalkinyl-gruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Nickel, wie Raney-Nickel, sowie Edelmetalle oder deren Derivate, z.B. Oxide, geeignet sind, wie Palladium oder Platinoxid, die gegebenenfalls auf Trägermaterialien, z.B. auf Kohle oder Calciumcarbonat, aufgezogen sein können. Die Hydrierung kann vorzugsweise bei Drucken zwischen etwa 1 und etwa 100 at und bei Temperaturen zwischen etwa -80° und etwa +200°C, vor allem zwischen Raumtemperatur und etwa 100°C, durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Die Erfindung betrifft insbesondere die in den Beispielen beschriebenen Verfahren.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens.

Salze von Verbindungen I können in an sich bekannter Weise in andere Salze von Verbindungen I umgewandelt werden.

Je nach Verfahrensweise bzw. Reaktionsbedingungen können die Verbindungen I mit salzbildenden, insbesondere basischen Eigenschaften, in freier Form oder in Form von Salzen erhalten werden.

Infolge der engen Beziehung zwischen der Verbindung I in freier Form und in Form ihrer Salze sind im Vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, z. B. zur Kristallisation verwendete, Lösungsmittel einschliessen.

Die Verbindungen I und ihre Salze können, je nach der Wahl der Ausgangsstoffe und Arbeitsweisen, in Form eines der möglichen Isomeren oder als Gemisch derselben, z.B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren oder Racemate aufgetrennt werden, beispielsweise durch fraktionierte Kristallisation. Erhaltene Enantiomerengemische, wie Racemate, lassen sich nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Überführung in diastereomere Salze, z.B. durch Umsetzung eines basischen Endstoffracemats mit einer optisch aktiven Säure, wie Carbonsäure, z.B. Wein- oder Äpfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsgedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen. Neue Ausgangsstoffe und Zwischenprodukte für die Herstellung der Verbindungen I, ihre Verwendung und ein Verfahren zu ihrer Herstellung bilden ebenfalls einen Gegenstand der Erfindung, wobei die Variablen $R_1$, $R_2$, $R_3$, p, q und X und die Ringe A und B die für die Verbindungen I angegebenen Bedeutungen haben.

Die Verbindungen I und ihre pharmazeutisch verwendbaren Salze können, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antihypertensiva, verwendet werden.

Die Erfindung betrifft daher gleichfalls pharmazeutische Präparate, die eine Verbindung I in freier Form oder in Form eines pharmazeutisch verwendbaren Salzes als Wirkstoff enthalten, sowie ein Verfahren zu ihrer Herstellung. Bei diesen pharmazeutischen Präparaten handelt es sich um solche zur enteralen, wie oralen, ferner rektalen oder parenteralen Verabreichung an Warmblüter, wobei der pharmakologische Wirkstoff allein oder zusammen mit üblichen pharmazeutischen Hilfsstoffen enthalten ist. Die pharmazeutischen Präparate enthalten z.B. von etwa 0,1 % bis 100 %, vorzugsweise von etwa 1 % bis etwa 60 %, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragantgummi, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzubsweise in geeigneten Müssigkeiten, wie fetten Ölen, Paraffinöl oder Müssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole und höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole und Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet, oder wässrige Injektiönssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffes kann von verschiedenen Faktoren, wie Applikationsweise, Warmblüter-Spezies, Alter und/oder individuellem Zustand, abhängen. Im Normalfall ist für einen etwa 75 kg schweren Patienten bei oraler Applikation eine ungefähre Tagesdosis von etwa 10 mg bis etwa 250 mg zu veranschlagen.

Die nachfolgenden Beispiele illustrieren die oben beschriebene Erfindung; sie sollen jedoch diese in ihrem Umfang in keiner Weise einschränken. Temperaturen sind in Grad Celsius angegeben.

Beispiel 1:

Eine Lösung von 8, 8 g (22,7 mmol) 3-Methoxycarbonylpropansäure-N-butyl-N-(2′-cyanobiphenyl-4-ylmethyl)-amid und 9,8 g (29,5 mmol) Tributylzinnazid in 100 ml o-Xylol wird 20 Stunden unter Rückfluss erhitzt. Dann wird das Reaktionsgemisch auf 20° abgekühlt und mit 100 ml 1 N-Kalilauge versetzt. Man rührt 2 Stunden

kräftig durch, trennt die wässrige Phase ab und stellt diese mit 2 N-Salzsäure sauer. Das sich abscheidende Harz wird mit Ethylacetat extrahiert. Das durch Eindampfen der Ethylacetatphase erhaltene Rohprodukt wird an 500 g Silicagel Mash-chromatographiert [Toluol/Isopropanol/Eisessig ( 170:30:2)]. Man erhält so das 3-Carboxypropansäure-N-butyl-N- [2'-(1H-tetrazol-5-yl)biphenyl-4-ylmethyl]-amid in Form eines hellbraunen Schaumes, der einen $R_f$-Wert von 0,20 [Toluol/Isopropanol/Eisessig (170:30:2)] aufweist.

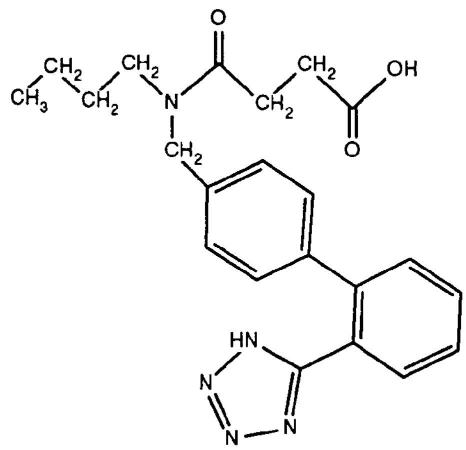

Das Ausgangsmaterial kann beispielsweise wie folgt hergestellt werden:

a) Eine Lösung von 20,0 g (73,5 mmol) 4-Brommethyl-2'-cyano-biphenyl (EP 253,310, Seite 159) in 150 ml Dioxan wird mit 36 ml (367 mmol) Butylamin versetzt und das Gemisch 3 Stunden unter Rückfluss erhitzt. Dann wird das Reaktionsgemisch im Vakuum eingedampft und der Rückstand zwischen 300 ml Diethylether und 100 ml Wasser verteilt. Die etherische Phase wird zweimal mit Wasser gewaschen, getrocknet und eingedampft. Das so erhaltene Oel wird an 500 g Silicagel Mash-chromatographiert [Toluol/Methanol (19:1)]. Die das Produkt enthaltenden Fraktionen werden eingedampft und liefern so das N-Butyl-N-(2'-cyanobiphenyl-4-ylmethyl)-amin in Form eines gelben Oels, das einen $R_f$-Wert von 0,34 [Toluol/Methanol (4:1)] aufweist.

b) Eine Lösung von 6,0 g (22,7 mmol) N-Butyl-N-(2'-cyanobiphenyl-4-ylmethyl)-amin und 13 ml Hünib-Base in 100 ml Ethylacetat wird tropfenweise unter Eiskühlung mit einer Lösung von 5,1 g (34 mmol) Bernsteinsäuremonomethylestermonochlorid in 20 ml Ethylacetat versetzt. Das Reaktionsgemisch wird 1 Stunde bei 0 bis 5° gerührt und dann mit 30 ml Wasser versetzt. Die organische Phase wird nacheinander mit 30 ml 2 N-Salzsäure, Wasser und gesättigter Kaliumhydrogencarbonatlösung gewaschen und betrocknet. Durch Abdampfen des Lösungsmittels erhält man das rohe 3-Methoxy-carbonylpropansäure-N-butyl-N-(2'-cyanobiphenyl-4-ylmethyl)-amid in Form eines Oels, das einen $R_f$-Wert von 0,32 [Toluol/Methanol (19:1)] besitzt.

Beispiel 2:

Eine Lösung von 4,0 g (9,8 mmol) 3-Methoxycarbonyl-3-methyl-butansäure-N-butyl-N-(2'-cyanobiphenyl-4-ylmethyl)-amid und 4,3 g (13 mmol) Tributylzinnazid in 80 ml o-Xylol wird 24 Stunden analog Beispiel I umgesetzt. Zur Hydrolyse wird das Reaktionsgemisch mit 50 ml 2 N-Kalilauge 4 Stunden bei 40 bis 50° verrührt. Die wässrige Phase wird abgetrennt und mit 2 N-Salzsäure sauer gestellt und das abgeschiedene Oel wird mit Ethylacetat extrahiert. Nach Waschen der Ethylacetatlösung mit Wasser, Trocknen über $MgSO_4$ und Eindampfen erhält man das 3-Carboxy-3-methyl-butansäure-N-butyl-N-[2'-(1H-tetrazol-5-yl)biphenyl-4-ylmethyl]-amid in Form eines farblosen Schaumes, der einen $R_f$-Wert von 0,25 [Toluol/Isopropanol/Eisessig (170:30:2)] aufweist.

Das Ausgangsmaterial kann beispielsweise wie folgt hergestellt werden:

a) Eine Lösung von 3,6 g (13,5 mmol) N-Butyl-N-(2'-cyanobiphenyl-4-ylmethyl)-amin und 1,75 g (13,5 mmol) 2,2-Dimethylbemsteinsäureanhydrid in 50 ml Dioxan wird 5 Stunden unter Rückfluss erhitzt. Nach Eindampfen des Reaktionsgemisches erhält man das rohe 3-Carboxy-3-methyl-butansäure-N-butyl-N-(2'-cyanobiphenyl-4-ylmethyl)-amid, das bei 125 bis 131° schmilzt und roh weiterverwendet wird.

b) Ein Gemisch aus 4,8 g (12,2 mmol) 3-Carboxy-3-methyl-butansäure-N-butyl-N-(2'-cyanobiphenyl-4-ylmethyl)-amid, 2,2 g (15,9 mmol) Kaliumcarbonat, 1,7 g (13,5 mmol) Dimethylsulfat und 50 ml N,N-Dimethylformamid wird 3 bis 4 Stunden bei 50 bis 60° Innentemperatur gerührt. Nach dem Abkühlen wird

das ungelöste Material abfiltriert, das Filtrat im Vakuum eingedampft und der Rückstand in Ethylacetat gelöst. Nach Waschen, Trocknen und Eindampfen der Lösung im Vakuum erhält man ein Rohprodukt, das durch Flash-chromatographie gereinigt wird [Toluol/Methanol (75:1)]. Das so erhaltene 3-Methoxycarbonyl-3-methyl-butansäure-N-butyl-N-(2′-cyanobiphenyl-4-ylmethyl)-amid liegt in Form eines gelblichen Oels vor, das einen $R_f$-Wert von 0,20 [Toluol/Isopropanol/Eisessig (170:30:2)] aufweist.

Beispiel 3:

Eine Lösung von 5,5 g (11,3 mmol) 4-Methoxycarbonyl-4-methyl-pentansäure-N-butyl-N-(2′-cyanobiphenyl-4-ylmethyl)-amid und 4,9 g (14,2 mmol) Tributylzinnazid in 50 ml o-Xylol wird analog Beispiel 2 umgesetzt und aufgearbeitet. Man erhält so das 4-Carboxy-4-methyl-pentansäure-N-butyl-N-[2′-(1H-tetrazol-5-yl)-biphenyl-4-ylmethyl]-amid in Form eines beige-farbenen Schaumes, der einen $R_f$-Wert von 0,34 [Toluol/Isopropanol/Eisessig (170:30:2)] aufweist.

Das Ausgangsmaterial kann beispielsweise wie folgt hergestellt werden: Zu einer Lösung von 3,0 g (11,3 mmol) N-Butyl-N-(2′-cyanobiphenyl-4-ylmethyl)-amin und 6,5 ml Hünib-Base in 50 ml Ethylacetat wird bei 0 bis 5° eine Lösung von 3,3 g (17 mmol) 4-Methoxycarbonyl-4-methyl-pentansäurechlorid in 20 ml Ethylacetat zugetropft. Das Reaktionsgemisch wird 1 Stunde gerührt und dann analog Beispiel 1 b) aufgearbeitet. Man erhält so das 4-Methoxycarbonyl-4-methyl-pentansäure-N-butylN-(2′-cyanobiphenyl-4-ylmethyl)-amid in Form eines Oels, das einen $R_f$-Wert von 0,27 [Toluol/Methanol (19:1)] aufweist und roh weiterverwendet wird.

Beispiel 4:

Eine Lösung von 3,45 g 3,3-Dimethyl-4-methoxycarbonyl-butansäure-N-(2′-cyanobiphenyl-4-ylmethyl)-N-propyl-amid und 11,3 g Tributylzinnazid in 100 ml Xylol wird 3 Stunden unter Rückfluss erhitzt. Dann gibt man 50 ml 2 N-Natronlauge zu und rührt das Gemisch 90 Minuten bei 60°. Die wässrige Phase wird nach dem Abkühlen angesäuert und einmal mit 100 ml sowie zweimal mit je 40 ml $CH_2Cl_2$ extrahiert. Die Dichlormethanphase liefert nach Trocknung über $Na_2SO_4$ und Entfernung der Lösungsmittel einen farblosen Schaum. Reinigung desselben durch Chromatographie an Kieselgel 60 (40-63 μm) mit $CH_2Cl_2/CH_3OH$ (95:5) als Laufmittel ergibt das reine 4-Carboxy-3,3-dimethyl-butansäure-N-propyl-N-[2′-(1H-tetrazol-5-yl)biphenyl-4-ylmethyl]-amid, das einen $R_f$-Wert von 0,44 [$CH_2Cl_2/CH_3OH$ (4:1)] aufweist.

Das Ausgangsmaterial kann beispielsweise wie folgt hergestellt werden:

a) 10,84 g 4-Brommethyl-2′-cyano-biphenyl werden bei Raumtemperatur mit 20 ml Propylamin vermischt. Das Gemisch wird 30 Minuten gerührt, wobei Erwärmung bis zur Rückflusstemperatur eintritt, dann mit 400 ml $CH_2Cl_2$ verdünnt und mit 200 ml $NaHCO_3$-Lösung gewaschen. Die organische Phase wird über $Na_2SO_4$ getrocknet und im Vakuum von den Lösungsmitteln befreit, woraufhin ein gelbes Oel zurückbleibt. Reinigung desselben durch Chromatographie an Kieselgel 60 (40-63 μm) mit $CH_2Cl_2/CH_3OH$ (98:2) als Laufmittel ergibt das reine N-(2′-Cyanobiphenyl-4-ylmethyl)-N-propyl-amin, das einen $R_f$-Wert von 0,15 [$CH_2Cl_2/CH_3OH$ (95:5)] aufweist.

b) Eine Lösung von 2,19 g N-(2′-Cyanobiphenyl-4-ylmethyl)-N-propyl-amin und 2,37 g 3,3-Dimethylglutarsäuremonomethylestermonochlorid in 100 ml $CH_2Cl_2$ wird mit 1,83 ml Triethylamin versetzt und das Gemisch bei Raumtemperatur 2 Stunden gerührt. Zweimalige Extraktion mit je 50 ml Wasser und Ausziehen der wässriben Phase mit 40 ml $CH_2Cl_2$ ergibt eine organische Phase, die nach üblichem Vorgehen nach Eindampfen das 3,3-Dimethyl-4-methoxycarbonyl-butansäure-N-(2′-cyanobiphenyl-4-ylmethyl)N-propyl-amid liefert, das einen $R_f$-Wert von 0,66 [$CH_2Cl_2/CH_3OH$ (95:5)] aufweist.

Beispiel 5:

Eine Lösung von 1,88 g 4-Methoxycarbonyl-4-methyl-pentansäure-N-(2′-cyanobiphenyl-4-ylmethyl)-N-propyl-amid und 6,0 g Tributylzinnazid in 50 ml Xylol wird 17 Stunden unter Rückfluss erhitzt. Das Gemisch wird dann eingedampft, der Rückstand in 25 ml 2 N-Natronlauge aufgenommen und das wässrige Gemisch dreimal mit je 50 ml Diethylether extrahiert. Die wässrige Phase wird angesäuert und einmal mit 50 ml sowie zweimal mitje 25 ml $CH_2Cl_2$ extrahiert. Das aus der $CH_2Cl_2$-Phase nach üblichem Vorgehen erhaltene Rohprodukt liefert nach Chromatographie an Kieselgel 60 (40-63 μm) mit $CH_2Cl_2/CH_3OH$ (95:5) als Laufmittel das reine 4-Methoxycarbonyl-4-methyl-pentansäure-N-propyl-N-[2′-(1H-tetrazol-5-yl)biphenyl-4-ylmethyl]-amid, das einen $R_f$-Wert von 0,59 [$CH_2Cl_2/CH_3OH$ (4:1)] aufweist.

Das Ausgangsmaterial kann beispielsweise wie folgt hergestellt werden: Eine Lösung von 2,50 g N-(2′-Cyanobiphenyl-4-ylmethyl)-N-propyl-amin und 2,09 ml Triethylamin in 100 ml $CH_2Cl_2$ wird mit 2,89 g 4-Methoxycarbonyl-4-methyl-pentansäurechlorid versetzt und das Gemisch 1 Stunde gerührt. Das Reaktionsgemisch

wird zweimal mit je 50 ml Wasser gewaschen und die vereinigten wässrigen Phasen werden mit 50 ml $CH_2Cl_2$ extrahiert. Das aus der organischen Phase nach üblichem Vorgehen erhaltene Rohprodukt wird durch Chromatographie an Kieselgel 60 (40-63 μm) mit $CH_2Cl_2/CH_3OH$ (98:2) als Laufmittel gereinigt und liefert so das reine 4-Methoxycarbonyl-4-methyl-pentansäure-N-(2′-cyanobiphenyl-4-ylmethyl)-N-propyl-amid, das einen $R_f$-Wert von 0,56 [$CH_2Cl_2/CH_3OH$ (95:5)] aufweist.

Beispiel 6:

Eine Lösung von 0,450 g 4-Methoxycarbonyl-4-methyl-pentansäure-N-propyl-N-[2′-(1H-tetrazol-5-yl)biphenyl-4-ylmethyl]-amid und 2,0 ml 2 N-Kalilauge in 10 ml Methanol wird 6 Stunden unter Rückfluss erhitzt. Das abgekühlte Gemisch wird angesäuert und eingedampft. Der Rückstand wird durch Chromatographie an Kieselgel 60 (40-63 μm) mit $CH_2Cl_2/CH_3OH$ (95:5) als Laufmittel gereinigt und liefert so das reine 4-Carboxy-4-methyl-pentansäure-N-propyl-N-[2′-(1H-tetrazol-5-yl)biphenyl-4-ylmethyl]-amid, das einen $R_f$-Wert von 0,40 [$CH_2Cl_2/CH_3OH$ (4:1)] aufweist.

Beispiel 7:

Eine Lösung von 2,93 g 4-Carboxy-4-ethyl-hexansäure-N-butyl-N-(2′-cyanobiphenyl-4-ylmethyl)-amid und 8,8 g Tributylzinnazid in 75 ml Xylol wird 23 Stunden unter Rückfluss erhitzt. Das abgekühlte Gemisch wird mit 50 ml 2 N-Natronlauge ausgezogen. Die wässrige Phase wird angesäuert und einmal mit 100 ml sowie zweimal mit je 30 ml $CH_2Cl_2$ extrahiert. Die vereinigten Dichlormethanphasen liefern nach üblicher Behandlung ein Rohprodukt, welches nach Chromatographie an Kieselgel 60 (40-63 μm) mit $CH_2Cl_2/CH_3OH$ (95:5) als Laufmittel das reine 4-Carboxy-4-ethylhexansäure-N-butyl-N- [2′-(1H-tetrazol-5-yl)biphenyl-4-ylmethyl]-amid, das einen $R_f$-Wert von 0,46 [$CH_2Cl_2/CH_3OH$ (4:1)] aufweist, ergibt.

Das Ausgangsmaterial kann beispielsweise wie folgt hergestellt werden: Eine Lösung von 3,2 g N-Butyl-N-(2′-cyanobiphenyl-4-ylmethyl)-amin und 3,09 g 2,2-Diethylglutarsäureanhydrid in 50 ml Dioxan wird 6 Stunden auf 115° (Badtemperatur) erhitzt. Nach dem Eindampfen des Reaktionsgemisches wird der Rückstand in 100 ml Ethylacetat aufgenommen und die Ethylacetatphase zweimal mit je 50 ml Wasser gewaschen, über $Na_2SO_4$ getrocknet und im Vakuum eingedampft. Chromatographie des Eindampfrückstands an Kieselgel 60 (40-63 μm) mit $CH_2Cl_2/CH_3OH$ (95:5) als Laufmittel ergibt das reine 4-Carboxy-4-ethyl-hexansäure-N-butyl-N-(2′-cyanobiphenyl-4-ylmethyl)-amid, das einen $R_f$-Wert von 0,72 [$CH_2Cl_2/CH_3OH$ (4:1)] aufweist.

Beispiel 8:

Analog Beispiel 4 wird, ausgehend von 4,04 g 2-Ethoxycarbonylbutansäure-N-butyl-N-(2′-cyanobiphenyl-4-ylmethyl)-amid, nach Chromatographie des Rohprodukts an Kieselgel 60 (40-63 μm) mit $CH_2Cl_2/CH_3OH$ (9:1) als Laufmittel das reine 2-Carboxybutansäure-N-butyl-N-[2′-(1H-tetrazol-5-yl)biphenyl-4-ylmethyl]-amid, das einen $R_f$-Wert von 0,56 [$CH_2Cl_2/CH_3OH$ (4:1)] aufweist, erhalten.

Das Ausgangsmaterial kann beispielsweise wie folgt hergestellt werden: Analog Beispiel 4b) wird, ausgehend von 2,64 g N-Butyl-N-(2′-cyanobiphenyl-4-ylmethyl)-amin und 2,68 g Ethylmalonsäuremonoethylestermonochlorid, das 2-Ethoxycarbonylbutansäure-N-butyl-N-(2′-cyanobiphenyl-4-ylmethyl)-amid, das einen $R_f$-Wert von 0,65 [$CH_2Cl_2/CH_3OH$ (95:5)] aufweist, erhalten.

Beispiel 9:

Analog Beispiel 4 wird, ausgehend von 4,20 g 4-Carboxy-4-ethyl-hexansäureN-(2′-cyanobiphenyl-4-ylmethyl)-N-propyl-amid, nach Chromatographie des Rohprodukts an Kieselbel 60 (40-63 μm) mit $CH_2Cl_2/CH_3OH$ (95:5) als Laufmittel das reine 4-Carboxy-4-ethyl-hexansäure-N-propyl-N-[2′-(1H-tetrazol-5-yl)biphenyl-4-ylmethyl]amid, das einen $R_f$-Wert von 0,58 [$CH_2Cl_2/CH_3OH$ (4:1)] aufweist, erhalten.

Das Ausgangsmaterial kann beispielsweise wie folgt hergestellt werden: Ein Gemisch aus 2,50 g N-(2′-Cyanobiphenyl-4-ylmethyl)-N-propyl-amin, 2,58 g 2,2-Diethylglutarsäureanhydrid und 50 ml Dioxan wird 1 Stunde unter Rückfluss erhitzt. Nach Eindampfen des Reaktionsgemisches wird der Rückstand in 100 ml Ethylacetat aufgenommen und die Ethylacetatphase zweimal mit je 50 ml Wasser gewaschen, über Natriumsulfat getrocknet und im Vakuum eingedampft. Das so erhaltene 4-Carboxy-4-ethyl-hexansäure-N-(2′-cyanobiphenyl-4-ylmethyl)-N-propyl-amid wird roh weiterverwendet.

Beispiel 10:

Analog Beispiel 4 wird, ausgehend von 4,19 g 4-Carboxy-3,3-tetramethylenbutansäure-N-(2′-cyanobiphenyl-4-ylmethyl)-N-propyl-amid, nach Chromatographie des Rohprodukts an Kieselgel 60 (40-63 μm) mit $CH_2Cl_2/CH_3OH$ (95:5) als Laufmittel das reine 4-Carboxy-3,3-tetramethylen-butansäure-N-propyl-N-[2′-(1H-tetrazol-5-yl)-biphenyl-4-ylmethyl] -amid, das einen $R_f$-Wert von 0,48 [$CH_2Cl_2/CH_3OH$ (4:1)] aufweist, erhalten.

Das Ausgangsmaterial kann beispielsweise wie folgt hergestellt werden:

Analog Beispiel 7a) werden 2,50 g N-(2′-Cyanobiphenyl-4-ylmethyl)-N-propyl-amin mit 2,02 g 3,3-Tetramethylenglutarsäureanhydrid umgesetzt. Nach extraktiver Aufarbeitung erhält man das 4-Carboxy-3,3-tetramethylen-butansäure-N-(2′-cyanobiphenyl-4-ylmethyl)-N-propyl-amid, das einen $R_f$-Wert von 0,80 [$CH_2Cl_2/CH_3OH$ (4:1)] aufweist.

Beispiel 11:

Analog Beispiel 10 kann man das 4-Carboxy-3,3-tetramethylen-butansäure-N-butyl-N-[2′-(1H-tetrazol-5-yl)biphenyl-4-ylmethyl]-amid, das einen $R_f$-Wert von 0,49 [$CH_2Cl_2/CH_3OH$ (4:1)] aufweist, erhalten.

Beispiel 12:

Analog Beispiel 10 kann man das 3-Carboxy-3,3-tetramethylen-propansäure-N-propyl-N-[2′-(1H-tetrazol-5-yl)biphenyl-4-ylmethyl]-amid, das bei 104 bis 109° schmilzt, erhalten.

Beispiel 13:

Analog Beispiel 10 kann man das 3-Carboxy-3,3-tetramethylen-propansäure-N-butyl-N-[2′-(1H-tetrazol-5-yl)biphenyl-4-ylmethyl]-amid, das bei 122 bis 127° schmilzt, erhalten.

Beispiel 14:

Analog Beispiel 9 kann man das 3-Carboxy-3-ethyl-pentansäure-N-propyl-N-[2′-(1H-tetrazol-5-yl)biphenyl-4-ylmethyl]-amid, das bei 105 bis 114° schmilzt, erhalten.

Beispiel 15:

In analoger Weise wie in einem der vorhergehenden Beispiele beschrieben kann man auch erhalten:

1. das 4-Carboxy-4,4-tetramethylen-butansäure-N-propyl-N-[2′-(1H-tetrazol-5-yl)biphenyl-4-ylmethyl]-amid;
2. das 4-Carboxy-4,4-tetramethylen-butansäure-N-butyl-N-[2′-(1H-tetrazol-5-yl)biphenyl-4-ylmethyl]-amid;
3. das 2-Carboxy-2-ethyl-butansäure-N-butyl-N-[2′-(1H-tetrazol-5-yl)biphenyl-4-ylmethyl]-amid;
4. das 2-Carboxy-2-ethyl-butansäure-N-propyl-N-[2′-(1H-tetrazol-5-yl)biphenyl4-ylmethyl]-amid;
5. das 2-Carboxybutansäure-N-propyl-N-[2′-(1H-tetrazol-5-yl)biphenyl-4-ylmethyl]-amid;
6. das 3-Carboxy-3-ethyl-pentansäure-N-butyl-N-[2′-(1H-tetrazol-5-yl)biphenyl4-ylmethyl]-amid;
7. das 3-Carboxy-3,3-pentamethylen-propansäure-N-propyl-N-[2′-(1H-tetrazol-5-yl)biphenyl-4-ylmethyl]-amid;
8. das 3-Carboxy-3,3-pentamethylen-propansäure-N-butyl-N-[2′-(1H-tetrazol-5-yl)biphenyl-4-ylmethyl]-amid;
9. das 5-Carboxy-5,5-tetramethylen-pentansäure-N-propyl-N-[2′-(1H-tetrazol-5-yl)biphenyl-4-ylmethyl]-amid;
10. das 5-Carboxy-5,5-tetramethylen-pentansäure-N-butyl-N-[2′-(1H-tetrazol-5-yl)biphenyl-4-ylmethyl]-amid;
11. das 3-Carboxy-2,2-tetramethylen-propansäure-N-propyl-N-[2′-(1H-tetrazol-5-yl)biphenyl-4-ylmethyl]-amid;
12. das 3-Carboxy-2,2-tetramethylen-propansäure-N-butyl-N-[2′-(1H-tetrazol-5-yl)biphenyl-4-ylmethyl]-amid;
13. das 2,2-Pentamethylen-2-trifluormethansulfonylamino-ethansäure-N-butyl-N-[2′-(1H-tetrazol-5-yl)-biphenyl-4-ylmethyl]-amid; und
14. das 2,2-Pentamethylen-2-trifluormethansulfonylamino-ethansäure-N-propyl-N-[2′-(1H-tetrazol-5-yl)-biphenyl-4-ylmethyl]-amid.

Beispiel 16:

Eine Lösung von 2,58 g 4-Carboxy-4,4-pentamethylen-butansäure-N-propyl-N-(2′-cyanobiphenyl-4-ylmethyl)-amid und 8,0 g Tributylzinnazid in 60 ml Xylol wird 16 Stunden unter Rückfluss erhitzt. Das abgekühlte

Gemisch wird mit 30 ml 2N Natronlauge 30 Minuten gerührt. Die wässrige Phase wird dreimal mit 50 ml Ether ausgezogen und daraufhin angesäuert und dreimal mit 50 ml $CH_2Cl_2$ extrahiert. Die vereinigten Dichlormethanphasen liefern nach üblicher Behandlung ein Rohprodukt, welches nach Chromatographie an Kieselgel 60 (40-63 µm) mit $CH_2Cl_2/CH_3OH$ (95:5) als Elutionsmittel 4-Carboxy-4,4-pentamethylen-butansäure-N-propyl-N-[2′-(1H-tetrazol-5-yl)-biphenyl-4-ylmethyl]-amid, das einen $R_f$-Wert von 0,48 [$CH_2Cl_2/CH_3OH$ (4:1)] aufweist, ergibt.

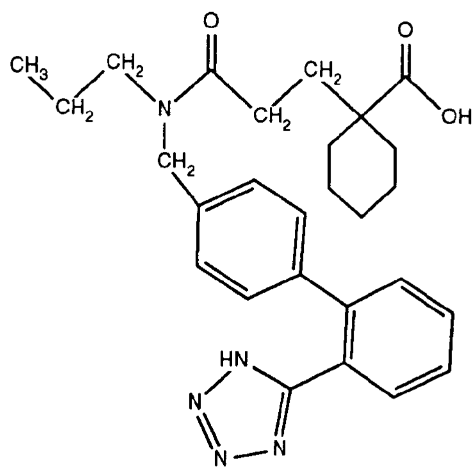

Das Ausgangsmaterial kann beispielsweise wie folgt hergestellt werden:

Eine Lösung von 1,37 g N-(2′-Cyanobiphenyl-4-ylmethyl)-N-propyl-amin und 1,20 g 2-Oxaspiro[5.5]undecan-1,3-dion in 30 ml Dioxan wird 3 Stunden unter Rückfluss erhitzt. Nach Entfernen des Lösungsmittels wird in 50 ml Essigester aufgenommen und zweimal mit 25 ml Wasser gewaschen. Die organische Phase liefert nach Trocknen und Eindampfen 4-Carboxy-4,4-pentamethylen-butansäure-N-propyl-N-(2′-cyanobiphenyl-4-ylmethyl)-amid.

Beispiel 17:

Analog Beispiel 16 wird, ausgehend von 2,63 g 4-Carboxy-4,4-pentamethylen-butansäure-N-butyl-N-(2′-cyanobiphenyl-4-ylmethyl)-amid und 7,80 g Tributylzinnazid, nach Chromatographie an Kieselgel 60 (40-63 mm) mit $CH_2Cl_2/CH_3OH$ (95:5) als Elutionsmittel 4-Carboxy-4,4-pentamethylen-butansäure-N-butyl-N-[2′-(1H-tetrazol5-yl)-biphenyl-4-ylmethyl]-amid, welches einen $R_f$-Wert von 0,32 [$CH_2Cl_2/CH_3OH$ (4:1)] aufweist, erhalten.

Das Ausgangsmaterial kann beispielsweise wie folgt hergestellt werden:

Analog Beispiel 16 werden 1,35 g N-Butyl-N-(2′-cyanobiphenyl-4-ylmethyl)-amin mit 1,20 g 2-Oxaspiro[5.5]undecan- 1,3-dion zu 4-Carboxy-4,4-pentamethylen-butansäure-N-butyl-N-(2′-cyanobiphenyl-4-ylmethyl)-amid umgesetzt.

Beispiel 18:

Eine Lösung von 2,76 g N-(2′-Cyanobiphenyl-4-ylmethyl)-N-propyl-isophthalsäure-mono-amid und 8,68 g Tributylzinnazid in 75 ml Xylol wird 2 Stunden unter Rückfluss erhitzt. Das abgekühlte Gemisch wird mit 50 ml 2N Natronlauge 1 Stunde bei Raumtemperatur gerührt, daraufhin angesäuert und mit 100 ml sowie zweimal 50 ml $CH_2Cl_2$ extrahiert. Die vereinigten Dichlormethanphasen liefern nach üblicher Behandlung ein Rohprodukt, welches nach Chromatographie an Kieselgel 60 (40-63 µm) mit $CH_2Cl_2/CH_3OH$ (9:1) als Elutionsmittel N-Propyl-N-[2′-(1H-tetrazol-5-yl)-biphenyl4-ylmethyl]-isophthalsäure-mono-amid, das einen $R_f$-Wert von 0, 10 [$CH_2Cl_2/CH_3OH$ (4:1)] aufweist, ergibt.

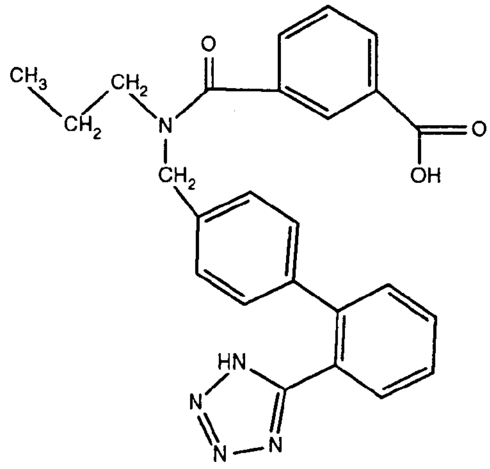

Das Ausgangsmaterial kann beispielsweise wie folgt hergestellt werden:

Eine Lösung von 2,50 g N-(2′-Cyanobiphenyl-4-ylmethyl)-N-propyl-amin, 2,98 g Isophthalsäure-monomethylester-mono-chlorid und 2, 10 ml Triethylamin in 100 ml $CH_2Cl_2$ wird 1 Stunde bei Raumtemperatur gerührt. Extraktion mit zweimal 50 ml Wasser und übliche Behandlung der organischen Phase liefert ein Rohprodukt, welches nach Chromatographie an Kieselgel 60 (40-63 μm) mit $CH_2Cl_2$ als Elutionsmittel N-(2′-Cyanobiphenyl-4-ylmethyl)-N-propyl-isophthalsäure-mono-amid, welches einen $R_f$-Wert von 0,61 [$CH_2Cl_2$/$CH_3OH$ (4:1)] aufweist, ergibt.

Beispiel 19:

Eine Lösung von 2,50 g 2-Carboxy-phenylessigsäure-N-butyl-N-(2′-cyanobiphenyl-4-ylmethyl)-amid und 6,50 g Tributylzinnazid in 50 ml Xylol wird 15 Stunden unter Rückfluss erhitzt. Das abgekühlte Gemisch wird mit 50 ml 2N Natronlauge 30 Minuten bei Raumtemperatur gerührt. Die wässrige Phase wird anbesäuert und mit dreimal 50 ml $CH_2Cl_2$ extrahiert. Die vereinigten Dichlormethanphasen liefern nach üblicher Behandlung ein Rohprodukt, welches nach Chromatographie an Kieselgel 60 (40-63 μm) mit $CH_2Cl_2$/$CH_3OH$ (97:3)) als Elutionsmittel 2-Carboxy-phenylessigsäure-N-butylN-[2′-(1H-tetrazol-5-yl)-bi phenyl-4-ylmethyl]-amid, das einen $R_f$-Wert von 0,37 [$CH_2Cl_2$/$CH_3OH$ (4:1)] aufweist, ergibt.

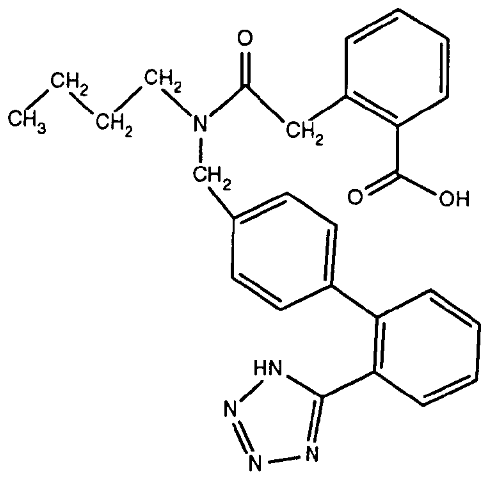

Das Ausgangsmaterial kann beispielsweise wie folgt hergestellt werden:

a) Eine Lösung von 2,64 g N-Butyl-N-(2′-cyanobiphenyl-4-ylmethyl)-amin und 2,43 g Homophthalsäureanhydrid in 50 ml Dioxan wird 1 Stunde unter Rückfluss erhitzt. Nach dem Entfernen des Lösungsmittels wird in 100 ml Essigester aufgenommen und mit zweimal 50 ml Wasser gewaschen. Die organische Phase liefert nach üblicher Behandlung ein Rohprodukt, welches aus $CH_2Cl_2$/Ether/Hexan 2-Carboxy-phenylessigsäure-N-butyl-N-(2′-cyanobiphenyl-4-ylmethyl)-amid, Smp. 137- 139°, ergibt.

Beispiel 20: N-Butyl-3-methyl-N-(2′-(1H-tetrazol-5-yl)biphenyl-4-ylmethyl]-2-(S)trifluormethansulfonylamino-butansäureamid

Analog Beispiel 1 erhält man aus 1,7 g (3,5 mmol) N-Butyl-N-(2′-cyanobiphenyl-4-ylmethyl)-3-methyl-2-(S)-trifluormethansulfonylamino-butansäureamid ein Rohprodukt, das an 150 g Kieselgel "flash"- chromatographiert wird (Elutionsmittel Ethylacetat/ Ethanol/conc. Ammoniak (6:3:1)). Die das Produkt enthaltenden Fraktionen ($R_f$-Wert 0,22) werden vereinigt und i.V. eingedampft. Der Eindampfrückstand wird zwischen Ethylacetat und 1-N Salzsäure verteilt. Die organische Phase wird abgetrennt, mit Sole gewaschen, getrocknet und eingedampft. Man erhält so die Titelverbindung als beigen Schaum (FAB-MS $MH^+$ = 539).

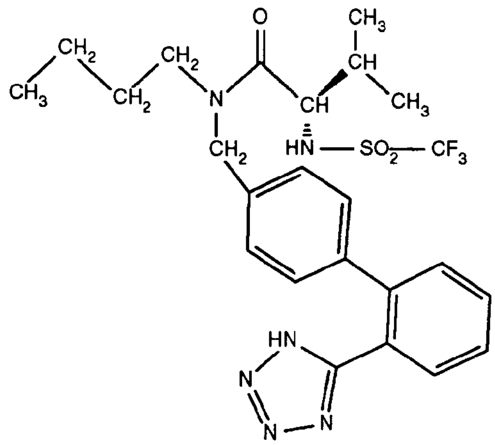

Das Ausgangsmaterial kann beispielsweise wie folgt hergestellt werden:

Analog Beispiel 21c) erhält man unter Verwendung von 1,3 g (3,5 mmol) 2-(S)-Amino-N-butyl-N- (2′-cyanobiphenyl-4-ylmethyl)-3-methyl-butansäureamid, 1,0 ml (5,8 mmol) Hünig-Base und 1,3 g (4,6 mmol) Trifluormethansulfonsäure-anhydrid das N-Butyl-N-(2′-cyanobiphenyl-4-ylmethyl)-3-methyl-2-(S)-trifluormethansulfonylaminobutansäureamid als rohes Oel, das ohne weitere Reinigung eingesetzt wird.

Beispiel 21: 2-(S)-Methansulfonylamino-3-methyl-butansäure-N-butyl-N-(2′-(1H-tetrazol-5-yl)biphenyl-4-ylmethyl]-amid

Analog Beispiel 20 erhält man aus 2,2 g (5 mmol) 2-(S)-Methansulfonylamino-3-methylbutansäure-N-butyl-N-(2′-cyanobiphenyl-4-ylmethyl)-amid und 2,5 g (7,5 mmol) Tributylzinnazid die Titelverbindung als hellen Schaum, der einen $R_f$-Wert von 0,45 [Toluol/Isopropanol/Eisessig (170:30:2)] aufweist.

Das Ausgangsmaterial kann beispielsweise wie folgt hergestellt werden:

a) Eine Lösung von 2,17 g (10 mmol) BOC-L-Valin und 1,1 ml (10 mmol) N-Methylmorpholin in 20 ml Dichlormethan wird auf -5° gekühlt. Unter Rühren werden 1,23 ml (10 mmol) Pivalinsäurechlorid bei -5° zugetropft. Die Lösung wird 2 Stunden bei 0° gerührt und hierauf mit einer Lösung von 2,64 g (10 mmol) N-Butyl-N-(2′-cyanobiphenyl-4-ylmethyl)-amin in 10 ml Dichlormethan tropfenweise versetzt. Es wird während 24 Stunden bei Raumtemperatur nachgerührt. Das Reaktionsgemisch wird mit 30 ml Dichlormethan verdünnt, nacheinander mit 1-n. Salzsäure (20 ml), gesättigter Kaliumbicarbonatlösung (10 ml) und Wasser gewaschen, getrocknet und i.V. eingedampft. Man erhält so das rohe N-Butyl-N-[2′-cyanobiphenyl-4-ylmethyl]-2-[(S)-t-butyloxycarbonylamino] -3-methylbutansäureamid als niederschmelzende Masse.

b) 4,7 g (10 mmol) rohes N-Butyl-N-[2′-cyanobiphenyl-4-ylmethyl]-2-[(S)-t-butyloxycarbonylamino]-3-methyl-butansäureamid wird in einem Gemisch von 30 ml Ameisensäure und 10 ml Dioxan gelöst und die Lösung 20 Stunden bei Raumtemperatur stehengelassen. Nach dem Eindampfen der Lösung i.V. wird der Rückstand zwischen Aethylacetat und 2 N Natronlauge verteilt, die organische Phase abgetrennt, mit Sole gewaschen, getrocknet und eingedampft. Man erhält so das 2-(S)-Amino-N-butyl-N-(2′-cyanobiphenyl-4-ylmethyl)-3-methyl-butansäureamid als gelbliches Oel, das ohne weitere Reinigung verwendet werden kann.

c) In eine Lösung von 1,8 g (5 mmol) 2-(S)-Amino-N-butyl-N-(2′-cyanobiphenyl-4-ylmethyl)-3-methylbutansäureamid und 1,4 ml (8 mmol) Hünigbase in 10 ml Dichlormethan wird unter Rühren eine Lösung von 0,75 g (6,5 mmol) Methansulfonsäurechlorid in 5 ml Dichlormethan getropft. Es wird noch weitere 5 Stunden bei Raumtemperatur gerührt, mit 50 ml Dichlormethan verdünnt und mit 20 ml 4-N Salzsäure extra-

hiert. Die Dichlormethan-Phase wird abgetrennt, nacheinander mit Wasser, 10 ml Bicarbonatlösung und Sole gewaschen, getrocknet und eingedampft. Man erhält so das rohe N-Butyl-N-(2′-cyanobiphenyl-4-ylmethyl)-2-(S)-methansulfonylamino-3-methylbutansäureamid als gelbliches Oel vom $R_f$-Wert 0,56 [Toluol/Isopropanol/Eisessig (170:30:2)], das ohne weitere Reinigung in die nächste Stufe eingesetzt wird.

Beispiel 22:

Tabletten, enthaltend je 50 mg Wirkstoff, z.B. 4-Carboxy-4-methylpentansäure-N-propyl-N-[2′-(1H-tetrazol-5-yl)biphenyl-4-ylmethyl]-amid, können wie folgt hergestellt werden:

| Zusammensetzung (für 10000 Tabletten): | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 23:

Lacktabletten, enthaltend je 100 mg Wirkstoff, z.B. 4-Carboxy-4-methylpentansäure-N-propyl-N-[2′-(1H-tetrazol-5-yl)biphenyl-4-ylmethyl]-amid, können wie folgt hergestellt werden:

| Zusammensetzung (für 1000 Tabletten): | |
|---|---|
| Wirkstoff | 100,00 g |
| Lactose | 100,00 g |
| Maisstärke | 70,00 g |
| Talk | 8,50 g |
| Calciumstearat | 1,50 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Dichlormethan lackiert (Endgewicht der Lacktablette: 283 mg).

Beispiel 24:

In analoger Weise wie in den Beispielen 22 und 23 beschrieben können auch Tabletten und Lacktabletten, enthaltend eine andere Verbindung I oder ein pharmazeutisch verwendbares Salz einer Verbindung I, z. B. gemäss einem der Beispiele 1 bis 21, hergestellt werden.

## Patentansprüche

1. Eine Verbindung der Formel

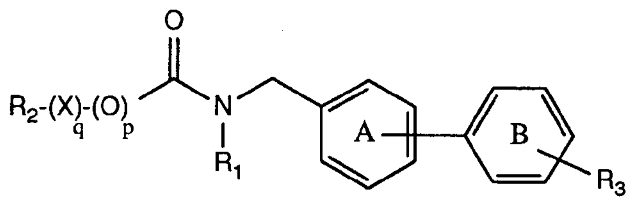

(I),

worin $R_1$ ein aliphatischer Kohlenwasserstoffrest, der gegebenenfalls in höherer als der β-Stellung zu dem in der Formel I gezeigten Stickstoffatom durch Halogen oder Hydroxy substituiert ist, ein cycloaliphatischer Kohlenwasserstoffrest oder ein araliphatischer Kohlenwasserstoffrest ist; $R_2$ 1H-Tetrazol-5-yl, Carboxy, verestertes Carboxy, amidiertes Carboxy, Amino, substituiertes Amio, Acylamino, Formyl, acetalisiertes Formyl, Hydroxy, verethertes Hydroxy, $S(O)_m$-R, wobei m 0, 1 oder 2 ist und R Wasserstoff oder ein aliphatischer Kohlenwasserstoffrest ist, Alkanoyl, Aminosulfonyl, N-substituiertes Aminosulfonyl, $PO_2H_2$, $PO_3H_2$ oder S-substituiertes Sulfonylamino bedeutet, $R_3$ Carboxy, 1H-Tetrazol-5-yl, $SO_3H$, $PO_2H_2$, $PO_3H_2$ oder Halogenalkansulfonylamino ist; X geradkettiges Alkylen, das gegebenenfalls aliphatisch überbrückt und/oder gegebenenfalls durch einen aliphatischen Kohlenwasserstoffrest substituiert ist, darstellt, oder für das Strukturelement der Formel $-X_1$-Ph-$X_2$- steht, wobei $X_1$ und $X_2$ unabhängig voneinander für eine Bindung oder für Alkylen stehen und Ph gegebenenfalls substituiertes Phenylen bedeutet; die Ringe A und B unabhängig voneinander unsubstituiert oder durch Halogen, Hydroxy, verethertes Hydroxy, $S(O)_m$-R, wobei m 0, 1 oder 2 ist und R Wasserstoff oder ein aliphatischer Kohlenwasserstoffrest ist, oder einen gegebenenfalls durch O unterbrochenen und/oder gegebenenfalls durch Halogen oder Hydroxy substituierten aliphatischen Kohlenwasserstoffrest substituiert sind; und entweder p und q beide für 1 stehen oder p 0 ist und q 0 oder 1 darstellt; in freier Form oder in Salzform.

2. Eine Verbindung gengäss Anspruch 1 der Formel I, worin $R_1$ Niederalkyl, Niederalkenyl, Niederalkinyl, Halogen in höherer als der β-Stellung zu dem in der Formel I gezeigten Stickstoffatom tragendes Halogen-niederalkyl, -niederalkenyl oder -niederalkinyl, Hydroxy in höherer als der β-Stellung zu dem in der Formel I gezeigten Stickstoffatom tragendes Hydroxy-niederalkyl, -niederalkenyl oder -niederalkinyl, Cycloniederalkyl, Cycloniederalkenyl, Phenylniederalkyl oder Phenyl-niederalkenyl oder -niederalkinyl ist; $R_2$ 1H-Tetrazol-5-yl, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, Amino, durch Niederalkyl, Niederalkenyl, Niederalkinyl oder Phenyl-niederalkyl, -niederalkenyl oder -niederalkinyl mono- oder unabhängig voneinander disubstituiertes oder durch Niederalkylen oder Niederalkylenoxyniederalkylen disubstituiertes Amino, durch Niederalkanoyl oder Benzoyl monosubstituiertes Amino, Formyl, Diniederalkoxymethyl, 1,3-Dioxacycloalk-2-yl, Hydroxy, Niederalkoxy, Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkanoyl, Aminosulfonyl, Aminosulfonyl, in welchem die Aminogruppe durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, $PO_2H_2$, $PO_3H_2$ oder Sulfonylamino, in welchem das Schwefelatom der Sulfonylaminogruppe durch Niederalkyl, Niederalkenyl, Niederalkinyl, Halogen-niederalkyl, -niederalkenyl oder -niederalkinyl, Phenyl-niederalkyl, -niederalkenyl oder -niederalkinyl oder Phenyl monosubstituiert ist, bedeutet, $R_3$ Carboxy, 1H-Tetrazol-5-yl, $SO_3H$, $PO_2H_2$, $PO_3H_2$ oder

Halogenniederalkansulfonylamino ist; X geradkettiges Niederalkylen, geradkettiges Niederalkylen, in welchem eine oder unabhängig voneinander zwei Methylengruppe(n) durch Cycloniederalk-1,1-ylen ersetzt ist (sind), geradkettiges Niederalkylen, das eine oder unabhängig voneinander zwei-, drei- oder vierfach durch Niederalkyl, Niederalkenyl oder Niederalkinyl substituiert ist, oder geradkettiges Niederalkylen, das ein- oder unabhängig voneinander zwei-, drei- oder vierfach durch Niederalkyl, Niederalkenyl oder Niederalkinyl substituiert ist und in welchem eine oder unabhängig voneinander zwei Methylengruppe(n) der in der entsprechenden unsubstituierten geradkettigen Niederalkylengruppe vorhandenen Methylengruppen durch Cycloniederalk-1,1-ylen ersetzt ist (sind), darstellt; die Ringe A und B unabhängig voneinander unsubstituiert oder durch Halogen, Hydroxy, Niederalkoxy,Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkyl, Niederalkenyl, Niederalkinyl, Halogen-niederalkyl, -niederalkenyl oder -niederalkinyl, Hydroxy-niederalkyl, -niederalkenyl oder -niederalkinyl, gegebenenfalls durch Halogen oder Hydroxy substituiertes Niederalkoxy-niederalkyl, -niederalkenyl oder -niederalkinyl oder gegebenenfalls durch Halogen oder Hydroxy substituiertes Niederalkenyloxy-niederalkyl, -niederalkenyl oder -niederalkinyl substituiert sind; und entweder p und q beide für 1 stehen oder p 0 ist und q 0 oder 1 darstell; wobei Phenyl in Phenylresten per se und in Phenylniederalkyl-, Phenylniederalkenyl-, Phenylniederalkinyl-, Benzoyl-, Phenoxy- und Benzyloxy-Gruppen jeweils unsubstituiertes oder ein- oder unabhängig voneinander mehrfach durch (einen) Substituenten ausgewählt aus der Gruppe, die aus Halogen, Hydroxy, Niederalkoxy, Niederalkyl und Trifluormethyl besteht, substituiertes Phenyl bedeutet; in freier Form oder in Salzform.

3. Eine Verbindung gemäss Anspruch 1 der Formel I, worin $R_1$ Niederalkyl, Niederalkenyl oder Cycloniederalkyl ist; $R_2$ 1H-Tetrazol-5-yl, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, Amino, durch Niederalkyl, Niederalkenyl, Niederalkinyl oder Phenyl-niederalkyl, -niederalkenyl oder -niederalkinyl mono- oder unabhängig voneinander disubstituiertes oder durch Niederalkylen oder Niederalkylenoxyniederalkylen disubstituiertes Amino, durch Niederalkanoyl oder Benzoyl monosubstituiertes Amino, Formyl, Diniederalkoxymethyl, 1,3-Dioxacycloalk-2-yl, Hydroxy, Niederalkoxy, Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkanoyl, Aminosulfonyl, Aminosulfonyl, in welchem die Aminogruppe durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, $PO_2H_2$, $PO_3H_2$ oder Sulfonylamino, in welchem das Schwefelatom der Sulfonylaminogruppe durch Niederalkyl, Niederalkenyl, Niederalkinyl, Halogen-niederalkyl, -niederalkenyl oder -niederalkinyl, Phenyl-niederalkyl, -niederalkenyl oder -niederalkinyl oder Phenyl monosubstituiert ist, bedeutet; $R_3$ Halogenniederalkansulfonylamino, Carboxy oder 1H-Tetrazol-5-yl ist; X geradkettiges Niederalkylen, geradkettiges Niederalkylen, in welchem eine Methylengruppe durch Cycloniederalk-1,1-ylen ersetzt ist, geradkettiges Niederalkylen, das ein- oder unabhängig voneinander zwei-, drei- oder vierfach durch Niederalkyl, Niederalkenyl oder Niederalkinyl substituiert ist, oder geradkettiges Niederalkylen, das ein- oder unabhängig voneinander zweifach durch Niederalkyl, Niederalkenyl oder Niederalkinyl substituiert ist und in welchem eine Methylengruppe der in der entsprechenden unsubstituierten geradkettigen Niederalkylengruppe vorhandenen Methylengruppen durch Cycloniederalk-1,1-ylen ersetzt ist, darstellt, die Ringe A und B unabhängig voneinander unsubstituiert oder durch Halogen, Hydroxy, Niederalkoxy, $S(O)_m$-R, wobei m 0, 1 oder 2 ist und R Wasserstoff oder Niederalkyl ist, Niederalkyl, Halogen-niederalkyl, Hydroxy-niederalkyl, gegebenenfalls durch Halogen oder Hydroxy substituiertes Niederalkoxy-niederalkyl oder gegebenenfalls durch Halogen oder Hydroxy substituiertes Niederalkenyloxy-niederalkyl substituiert sind; und entweder p und q beide für 1 stehen oder p 0 ist und q 0 oder 1 darstellt; wobei Phenyl in Phenylresten per se und in Phenylniederalkyl-, Phenylniederalkenyl-, Phenylniederalkinyl-, Benzoyl-, Phenoxyund Benzyloxy-Gruppen jeweils unsubstituiertes oder ein- oder unabhängig voneinander zweifach durch (einen) Substituenten ausgewählt aus der Gruppe, die aus Halogen, Hydroxy, Niederalkoxy, Niederalkyl und Trifluormethyl besteht, substituiertes Phenyl bedeutet; in freier Form oder in Salzform.

4. Eine Verbindung gemäss Anspruch 1 der Formel I, worin $R_1$ Niederalkyl oder Niederalkenyl ist; $R_2$ 1H-Tetrazol-5-yl, Carboxy, Carboxy, welches durch einen Alkohol verestert ist, dessen alkoholische Hydroxyfunktion an Niederalkyl oder Niederalkoxyniederalkyl gebunden ist, Carbamoyl, dessen Aminogruppe

unsubstituiert oder durch Niederalkyl oder Phenyl-niederalkyl mono- oder unabhängig voneinander disubstituiert oder durch Niederalkylen oder Niederalkylenoxyniederalkylen disubstituiert ist, Amino, durch Niederalkyl oder Phenyl-niederalkyl mono- oder unabhängig voneinander disubstituiertes oder durch Niederalkylen oder Niederalkylenoxyniederalkylen disubstituiertes Amino, durch Niederalkanoyl monosubstituiertes Amino, Hydroxy, Niederalkoxy, Phenoxy, Benzyloxy, $S(O)_m$-R, wobei m 0, 1 oder 2 ist und R Wasserstoff oder Niederalkyl ist, Niederalkanoyl, Aminosulfonyl, $PO_2H_2$, $PO_3H_2$ oder Sulfonylamino, in welchem das Schwefelatom der Sulfonylaminogruppe durch Niederalkyl, Halogenniederalkyl, Phenylniederalkyl oder Phenyl monosubstituiert ist, bedeutet; $R_3$ Carboxy oder 1H-Tetrazol-5-yl ist; X geradkettiges Niederalkylen, geradkettiges Niederalkylen, in welchem eine Methylengruppe durch Cycloniederalk-1,1-ylen ersetzt ist, oder geradkettiges Niederalkylen, das ein- oder unabhängig voneinander zweifach durch Niederalkyl substituiert ist, darstellt; die Ringe A und B unabhängig voneinander unsubstituiert oder durch Halogen, Hydroxy, Niederalkoxy, $S(O)_m$-R, wobei m 0, 1 oder 2 ist und R Wasserstoff oder Niederalkyl ist, oder Niederalkyl substituiert sind; p 0 ist, q 0 oder 1 darstellt; und der Ring B in 4-Position des Ringes A gebunden ist; wobei Phenyl in Phenylniederalkyl-, Phenoxy- und Benzyloxy-Gruppen jeweils unsubstituiertes oder einfach durch einen Substituenten ausgewählt aus der Gruppe, die aus Halogen, Niederalkoxy, Niederalkyl und Trifluormethyl besteht, substituiertes Phenyl bedeutet; in freier Form oder in Salzform.

5. Eine Verbindung gemäss einem der Ansprüche 1-4 der Formel I, worin X für das Strukturelement der Formel $-X_1$-Ph-$X_2$- steht, wobei $X_1$ und $X_2$ unabhängig voneinander für eine Bindung oder für $C_1$-$C_7$-Alklyen stehen und Ph Phenylen bedeutet; wobei Phenylen unsubstituiertes oder einfach durch einen Substituenten ausgewählt aus der Gruppe, die aus Halogen, Niederalkoxy, Niederalkyl und Trifluormethyl besteht, substituiertes Phenylen bedeutet; in freier Form oder in Salzform.

6. Eine Verbindung gemäss einem der Ansprüche 1-5 der Formel I, worin der Ring B in 4-Position des Ringes A gebunden ist; wobei Phenyl in Phenylniederalkyl-, Phenoxy- und Benzyloxy-Gruppen jeweils unsubstituiertes oder einfach durch einen Substituenten ausgewählt aus der Gruppe, die aus Halogen, Niederalkoxy, Niederalkyl und Trifluormethyl besteht, substituiertes Phenyl bedeutet; in freier Form oder in Salzform.

7. Eine Verbindung gemäss Anspruch 1 der Formel I, worin $R_1$ $C_1$-$C_4$-Alkyl oder $C_3$-$C_5$-Alkenyl ist, $R_2$ 1H-Tetrazol-5-yl, Carboxy, $C_1$-$C_4$-Alkoxycarbonyl, Carbamoyl, N-$C_1$-$C_4$-Alkylcarbamoyl, N,N-Di-$C_1$-$C_4$-alkylcarbamoyl, $C_1$-$C_4$-Alkanoyl, Aminosulfonyl oder Sulfonylamino, in welchem das Schwefelatom der Sulfonylaminogruppe durch $C_1$-$C_4$-Alkyl monosubstituiert ist, bedeutet; $R_3$ Carboxy oder 1H-Tetrazol-5-yl ist; X geradkettiges $C_1$-$C_7$-Alkylen, in welchem eine Methylengruppe durch $C_3$-$C_7$-Cycloalk-1,1-ylen ersetzt ist, oder geradkettiges $C_1$-$C_7$-Alkylen, das ein- oder unabhängig voneinander zweifach durch $C_1$-$C_4$-Alkyl substituiert ist, darstellt; die Ringe A und B unsubstituiert sind, p 0 ist, q 1 darstellt; der Ring B in 4-Position des Ringes A gebunden ist; und $R_3$ in einer 2-Position des Ringes B gebunden ist; in freier Form oder in Salzform.

8. Eine Verbindung gemäss Anspruch 1 der Formel I, worin $R_1$ $C_1$-$C_4$-Alkyl oder $C_3$-$C_5$-Alkenyl ist, $R_2$ 1H-Tetrazol-5-yl, Carboxy, $C_1$-$C_4$-Alkoxycarbonyl, Carbamoyl, N-$C_1$-$C_4$-Alkylcarbamoyl, N,N-Di-$C_1$-$C_4$-alkylcarbamoyl, $C_1$-$C_4$-Alkanoyl, Aminosulfonyl oder Sulfonylamino, in welchem das Schwefelatom der Sulfonylaminogruppe durch $C_1$-$C_4$-Alkyl monosubstituiert ist, bedeutet; $R_3$ Carboxy oder 1H-Tetrazol-5-yl ist; X für das Strukturelement der Formel $-X_1$-Ph-$X_2$- steht, wobei $X_1$ und $X_2$ unabhängig voneinander für eine Bindung oder für $C_1$-$C_3$-Alklyen stehen und Ph Phenylen bedeutet; die Ringe A und B unsubstituiert sind, p 0 ist, q 1 darstellt; der Ring B in 4-Position des Ringes A gebunden ist; und $R_3$ in einer 2-Position des Ringes B gebunden ist; in freier Form oder in Salzform.

9. Eine Verbindung gemäss Anspruch 1 der Formel I, worin $R_1$ $C_1$-$C_4$-Alkyl ist, $R_2$ Carboxy, $C_1$-$C_4$-Alkoxycarbonyl oder Carbamoyl bedeutet, $R_3$ 1H-Tetrazol-5-yl ist, X geradkettiges $C_1$-$C_7$-Alkylen, in welchem eine Methylengruppe durch $C_3$-$C_7$-Cycloalk-1,1-ylen ersetzt ist, oder geradkettiges $C_1$-$C_7$-Alkylen, das ein- oder unabhängig voneinander zweifach durch $C_1$-$C_4$-Alkyl substituiert ist, darstellt, die Ringe A und B unsubstituiert sind, p 0 ist, q 1 darstellt, der Ring B in 4-Position des Ringes A gebunden ist und $R_3$ in einer 2-Position des Ringes B gebunden ist, in freier Form oder in Salzform.

10. Eine Verbindung gemäss Anspruch 1 der Formel I, worin $R_1$ $C_1$-$C_4$-Alkyl ist, $R_2$ Carboxy bedeutet, $R_3$ 1H-Tetrazol-5-yl ist, X geradkettiges $C_1$-$C_7$-Alkylen, in welchem eine Methylengruppe durch $C_3$-$C_7$-Cycloalk-

1,1-ylen ersetzt ist, oder geradkettiges $C_1$-$C_7$-Alkylen, das ein- oder unabhängig voneinander zweifach durch $C_1$-$C_4$-Alkyl substituiert ist, darstellt, die Ringe A und B unsubstituiert sind, p 0 ist, q 1 darstellt, der Ring B in 4-Position des Ringes A gebunden ist und $R_3$ in einer 2-Position des Riges B gebunden ist, in freier Form oder in Salzform.

**11.** Eine Verbindung gemäss Anspruch 1 der Formel I, worin $R_1$ $C_3$-$C_4$-Alkyl ist; $R_2$ Carboxy bedeutet; $R_3$ 1H-Tetrazol-5-yl ist; X 2-Methyl-but- 1,3-ylen ist oder 1,3-Propylen bedeutet, in welchem die ω-Methylengruppe durch Cyclohex-1,1-ylen ersetzt ist; die Ringe A und B unsubstituiert sind; p 0 ist; q 1 darstellt; der Ring B in 4-Position des Ringes A gebunden ist; und $R_3$ in einer 2-Position des Ringes B gebunden ist; in freier Form oder in Salzform.

**12.** Eine Verbindung ausgewählt aus
3-Carboxypropansäure-N-butyl-N-[2′-(1H-tetrazol-5-yl)biphenyl-4-ylmethyl]-amid, in freier Form oder in Salzform;
3-Carboxy-3-methyl-butansäure-N-butyl-N-[2′-(1H-tetrazol-5-yl)biphenyl-4-ylmethyl]amid, in freier Form oder in Salzform;
4-Carboxy-4-methyl-pentansäure-N-butyl-N-[2′-(1H-tetrazol-5-yl)biphenyl-4-ylmethyl]amid, in freier Form oder in Salzform;
4-Carboxy-3,3-dimethyl-butansäure-N-propyl-N-[2′-(1H-tetrazol-5-yl)biphenyl-4-ylmethyl]-amid, in freier Form oder in Salzform;
4-Methoxycarbonyl-4-methyl-pentansäure-N-propyl-N-[2′-(1H-tetrazol-5-yl)biphenyl-4-ylmethyl]-amid, in freier Form oder in Salzform;
4-Carboxy-4-methyl-pentansäure-N-propyl-N-[2′-(1H-tetrazol-5-yl)biphenyl-4-ylmethyl]-amid, in freier Form oder in Salzform;
4-Carboxy-4-ethyl-hexansäure-N-butyl-N-[2′-(1H-tetrazol-5-yl)biphenyl-4-ylmethyl]amid, in freier Form oder in Salzform;
2-Carboxybutansäure-N-butyl-N- [2′-(1H-tetrazol-5-yl)biphenyl-4-ylmethyl]-amid, in freier Form oder in Salzform;
4-Carboxy-4-ethyl-hexansäure-N-propyl-N-[2′-(1H-tetrazol-5-yl)biphenyl-4-ylmethyl]amid, in freier Form oder in Salzform;
4-Carboxy-3,3-tetramethylen-butansäure-N-propyl-N-[2′-(1H-tetrazol-5-yl)biphenyl-4-ylmethyl] -amid, in freier Form oder in Salzform;
4-Carboxy-3,3-tetramethylen-butansäure-N-butyl-N-[2′-(1H-tetrazol-5-yl)biphenyl-4-ylmethyl]-amid, in freier Form oder in Salzform;
3-Carboxy-3,3-tetramethylen-propansäure-N-propyl-N-[2′-(1H-tetrazol-5-yl)biphenyl-4-ylmethyl]-amid, in freier Form oder in Salzform;
3-Carboxy-3,3-tetramethylen-propansäure-N-butyl-N-[2′-(1H-tetrazol-5-yl)biphenyl-4-ylmethyl]-amid, in freier Form oder in Salzform;
3-Carboxy-3-ethyl-pentansäure-N-propyl-N-[2′-(1H-tetrazol-5-yl)biphenyl-4-ylmethyl]amid, in freier Form oder in Salzform;
4-Carboxy-4,4-tetramethylen-butansäure-N-propyl-N-[2′-(1H-tetrazol-5-yl)biphenyl-4-ylmethyl]-amid, in freier Form oder in Salzform;
4-Carboxy-4,4-tetramethylen-butansäure-N-butyl-N-[2′-(1H-tetrazol-5-yl)biphenyl-4-ylmethyl]-amid, in freier Form oder in Salzform;
2-Carboxy-2-ethyl-butansäure-N-butyl-N-[2′-(1H-tetrazol-5-yl)biphenyl-4-ylmethyl]amid, in freier Form oder in Salzform;
2-Carboxy-2-ethyl-butansäure-N-propyl-N-[2′-(1H-tetrazol-5-yl)biphenyl-4-ylmethyl]amid, in freier Form oder in Salzform;
2-Carboxybutansäure-N-propyl-N-[2′-(1H-tetrazol-5-yl)biphenyl-4-ylmethyl]-amid, in freier Form oder in Salzform;
3-Carboxy-3-ethyl-pentansäure-N-butyl-N-[2′-(1H-tetrazol-5-yl)biphenyl-4-ylmethyl]amid, in freier Form oder in Salzform;
3-Carboxy-3,3-pentamethylen-propansäure-N-propyl-N-[2′-(1H-tetrazol-5-yl)biphenyl-4-ylmethyl]-amid, in freier Form oder in Salzform;
3-Carboxy-3,3-pentamethylen-propansäure-N-butyl-N-[2′-(1H-tetrazol-5-yl)biphenyl-4-ylmethyl]-amid, in freier Form oder in Salzform;
5-Carboxy-5,5-tetramethylen-pentansäure-N-propyl-N-[2′-(1H-tetrazol-5-yl)biphenyl-4-ylmethyl]-amid, in freier Form oder in Salzform;

5-Carboxy-5,5-tetramethylen-pentansäure-N-butyl-N-[2′-(1H-tetrazol-5-yl)biphenyl-4-ylmethyl]-amid, in freier Form oder in Salzform;
3-Carboxy-2,2-tetramethylen-propansäure-N-propyl-N- [2′-(1H-tetrazol-5-yl)biphenyl-4-ylmethyl]-amid, in freier Form oder in Salzform;
3-Carboxy-2,2-tetramethylen-propansäure-N-butyl-N-[2′-(1H-tetrazol-5-yl)biphenyl-4-ylmethyl]-amid, in freier Form oder in Salzform;
2,2-Pentamethylen-2-trifluormethansulfonylamino-ethansäure-N-butyl-N-[2′-(1H-tetrazol-5-yl)biphenyl-4-ylmethyl]-amid,in freier Form oder in Salzform; und
2,2-Pentamethylen-2-trifluormethansulfonylamino-ethansäure-N-propyl-N-[2′-(1H-tetrazol-5-yl)biphenyl -4-ylmethyl]-amid, in freier Form oder in Salzform.

**13.** Eine Verbindung ausgewählt aus
4-Carboxy-4,4-pentamethylen-butansäure-N-propyl-N-[2′-(1H-tetrazol-5-yl)-biphenyl-4-ylmethyl] -amid;
4-Carboxy-4,4-pentamethylen-butansäure-N-butyl-N-[2′-(1H-tetrazol-5-yl)-biphenyl-4-ylmethyl] -amid;
N-Propyl-N-[2′-(1H-tetrazol-5-yl)-biphenyl-4-ylmethyl]-isophthalsäure-mono-amid;
2-Carboxy-phenylessigsäure-N-butyl-N- [2′-(1H-tetrazol-5-yl)-biphenyl-4-ylmethyl]amid;
N-Butyl-3-methyl-N-[2′-(1H-tetrazol-5-yl)biphenyl-4-ylmethyl]-2-(S)-trifluormethansulfonylamino-butans äureamid; und
2-(S)-Methansulfonylamino-3-methyl-butansäure-N-butyl-N-[2′-(1H-tetrazol-5-yl)biphenyl-4-ylmethyl]-amid; jeweils in freier Form oder in Salzform.

**14.** Eine Verbindung gemäss einem der Ansprüche 1 bis 13 in freier Form oder in Form eines pharmazeutisch verwendbaren Salzes, zur Anwendung in einem Verfahren zur therapeutischen Behandlung des menschlichen oder tierischen Körpers.

**15.** Eine Verbindung gemäss einem der Ansprüche 1 bis 14, in freier Form oder in Form eines pharmazeutisch verwendbaren Salzes, zur Anwendung als Antihypertensivum.

**16.** Ein pharmazeutisches Präparat, als Wirkstoff enthaltend eine Verbindung gemäss einem der Ansprüche 1 bis 15 in freier Form oder in Form eines pharmazeutisch verwendbaren Salzes, gegebenenfalls neben üblichen pharmazeutischen Hilfsstoffen.

**17.** Ein antihypertensiv wirksames pharmazeutisches Präparat gemäss Anspruch 16.

**18.** Verfahren zur Herstellung einer Verbindung gemäss einem der Ansprüche 1 bis 13 der Formel I, in freier Form oder in Salzform, dadurch gekennzeichnet, dass man
a) in einer Verbindung der Formel

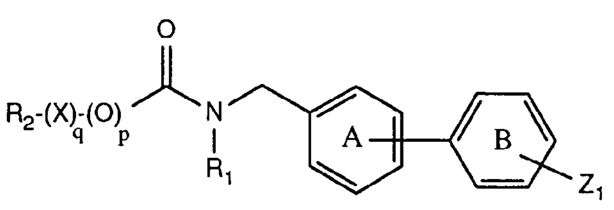

(II),

worin $Z_1$ einen in $R_3$ überführbaren Rest darstellt, oder in einem Salz davon $Z_1$ in $R_3$ überführt oder
b) eine Verbindung der Formel

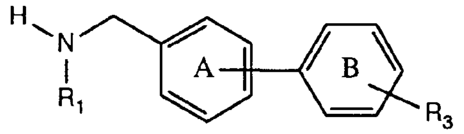

(III)

oder ein Salz davon mit einer Verbindung der Formel

$$R_2'\text{-}(X)_q\text{-}(O)_p\text{-}C(=O)\text{-}Z_2 \qquad (IV)$$

worin entweder $C(=O)$-$Z_2$ eine Carboxygruppe oder ein reaktionsfähiges Derivat davon darstellt und $R_2'$

ein Rest $R_2$ ist oder worin $R_2'$ und C(=O)-$Z_2$ gemeinsam die Gruppe -C(=O)-O-C(=O)- bilden , oder einem Salz davon umsetzt und jeweils, wenn erwünscht, eine verfahrensgengäss oder auf andere Weise erhältliche Verbindung I, in freier Form oder in Salzform, in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

**19.** Verwendung einer Verbindung gemäss einem der Ansprüche 1 bis 15, in freier Form oder in Form eines pharmazeutisch verwendbaren Salzes, zur Herstellung eines pharmazeutischen Präparats zur Behandlung von Bluthochdruck und/oder Herzinsuffizienz.

**Patentansprüche für folgenden Vertragsstaat : GR**

**1.** Verfahren zur Herstellung einer Verbindung der Formel

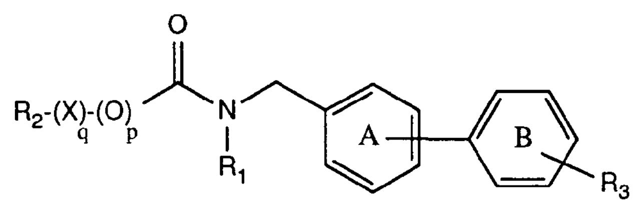

(I),

worin $R_1$ ein aliphatischer Kohlenwasserstoffrest, der gegebenenfalls in höherer als der β-Stellung zu dem in der Formel I gezeigten Stickstoffatom durch Halogen oder Hydroxy substituiert ist, ein cycloaliphatischer Kohlenwasserstoffrest oder ein araliphatischer Kohlenwasserstoffrest ist; $R_2$ 1H-Tetrazol-5-yl, Carboxy, verestertes Carboxy, amidiertes Carboxy, Amino, substituiertes Amino, Acylamino, Formyl, acetalisiertes Formyl, Hydroxy, verethertes Hydroxy, $S(O)_m$-R, wobei m 0, 1 oder 2 ist und R Wasserstoff oder ein aliphatischer Kohlenwasserstoffrest ist, Alkanoyl, Aminosulfonyl, N-substituiertes Aminosulfonyl, $PO_2H_2$, $PO_3H_2$ oder S-substituiertes Sulfonylamino bedeutet, $R_3$ Carboxy, 1H-Tetrazol-5-yl, $SO_3H$, $PO_2H_2$, $PO_3H_2$ oder Halogenalkansulfonylamino ist; X geradkettiges Alkylen, das gegebenenfalls aliphatisch überbrückt und/oder gegebenenfalls durch einen aliphatischen Kohlenwasserstoffrest substituiert ist, darstellt, oder für das Strukturelement der Formel -$X_1$-Ph-$X_2$- steht, wobei $X_1$ und $X_2$ unabhängig voneinander für eine Bindung oder für Alkylen stehen und Ph gegebenenfalls substituiertes Phenylen bedeutet; die Ringe A und B unabhängig voneinander unsubstituiert oder durch Halogen, Hydroxy, verethertes Hydroxy, $S(O)_m$-R, wobei m 0, 1 oder 2 ist und R Wasserstoff oder ein aliphatischer Kohlenwasserstoffrest ist, oder einen gegebenenfalls durch O unterbrochenen und/oder gegebenenfalls durch Halogen oder Hydroxy substituierten aliphatischen Kohlenwasserstoffrest substituiert sind; und entweder p und q beide für 1 stehen oder p 0 ist und q 0 oder 1 darstellt; in freier Form oder in Salzform;
dadurch gekennzeichnet, dass man
a) in einer Verbindung der Formel

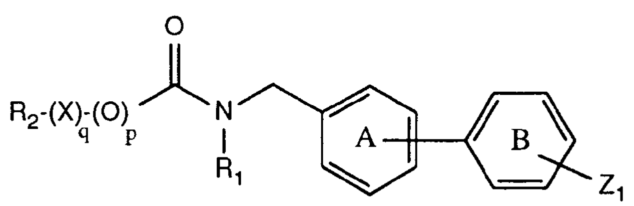

(II),

worin $Z_1$ einen in $R_3$ überführbaren Rest darstellt, oder in einem Salz davon $Z_1$ in $R_3$ überführt oder
b) eine Verbindung der Formel

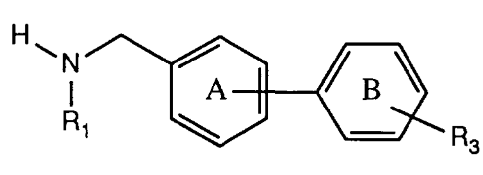

(III)

oder ein Salz davon mit einer Verbindung der Formel

$$R_2'-(X)_q-(O)_p-C(=O)-Z_2 \quad (IV),$$

worin entweder $C(=O)-Z_2$ eine Carboxygruppe oder ein reaktionsfähiges Derivat davon darstellt und $R_2'$ ein Rest $R_2$ ist oder worin $R_2'$ und $C(=O)-Z_2$ gemeinsam die Gruppe -C(=O)-O-C(=O)- bilden , oder einem Salz davon umsetzt und jeweils, wenn erwünscht, eine verfahrensgemäss oder auf andere Weise erhältliche Verbindung I, in freier Form oder in Salzform, in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

2. Verfahren nach Anspruch 1 zur Herstellung einer Verbindung der Formel I, worin $R_1$ Niederalkyl, Niederalkenyl, Niederalkinyl, Halogen in höherer als der β-Stellung zu dem in der Formel I gezeigten Stickstoffatom tragendes Halogen-niederalkyl, -niederalkenyl oder -niederalkinyl, Hydroxy in höherer als der β-Stellung zu dem in der Formel I gezeigten Stickstoffatom tragendes Hydroxy-niederalkyl, -niederalkenyl oder -niederalkinyl, Cycloniederalkyl, Cycloniederalkenyl, Phenylniederalkyl oder Phenyl-niederalkenyl oder -niederalkinyl ist; $R_2$ 1H-Tetrazol-5-yl, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, Amino, durch Niederalkyl, Niederalkenyl, Niederalkinyl oder Phenyl-niederalkyl, -niederalkenyl oder -niederalkinyl mono- oder unabhängig voneinander disubstituiertes oder durch Niederalkylen oder Niederalkylenoxyniederalkylen disubstituiertes Amino, durch Niederalkanoyl oder Benzoyl monosubstituiertes Amino, Formyl, Diniederalkoxymethyl, 1,3-Dioxacycloalk-2-yl, Hydroxy, Niederalkoxy, Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkanoyl, Aminosulfonyl, Aminosulfonyl, in welchem die Aminogruppe durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, $PO_2H_2$, $PO_3H_2$ oder Sulfonylamino, in welchem das Schwefelatom der Sulfonylaminogruppe durch Niederalkyl, Niederalkenyl, Niederalkinyl, Halogen-niederalkyl, -niederalkenyl oder -niederalkinyl, Phenyl-niederalkyl, -niederalkenyl oder -niederalkinyl oder Phenyl monosubstituiert ist, bedeutet, $R_3$ Carboxy, 1H-Tetrazol-5-yl, $SO_3H$, $PO_2H_2$, $PO_3H_2$ oder Halogenniederalkansulfonylamino ist; X geradkettiges Niederalkylen, geradkettiges Niederalkylen, in welchem eine oder unabhängig voneinander zwei Methylengruppe(n) durch Cycloniederalk-1,1-ylen ersetzt ist (sind), geradkettiges Niederalkylen, das ein- oder unabhängig voneinander zwei-, drei- oder vierfach durch Niederalkyl, Niederalkenyl oder Niederalkinyl substituiert ist, oder geradkettiges Niederalkylen, das ein- oder unabhängig voneinander zwei-, drei- oder vierfach durch Niederalkyl, Niederalkenyl oder Niederalkinyl substituiert ist und in welchem eine oder unabhängig voneinander zwei Methylengruppe(n) der in der entsprechenden unsubstituierten geradkettigen Niederalkylengruppe vorhandenen Methylengruppen durch Cycloniederalk-1,1-ylen ersetzt ist (sind), darstellt; die Ringe A und B unabhängig voneinander unsubstituiert oder durch Halogen, Hydroxy, Niederalkoxy, Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkyl, Niederalkenyl, Niederalkinyl, Halogen-niederalkyl, -niederalkenyl oder -niederalkinyl, Hydroxy-niederalkyl, -niederalkenyl oder -niederalkinyl, gegebenenfalls durch Halogen oder Hydroxy substituiertes Niederalkoxy-niederalkyl, -niederalkenyl oder -niederalkinyl oder gegebenenfalls durch Halogen oder Hydroxy substituiertes Niederalkenyloxy-niederalkyl, -niederalkenyl oder -niederalkinyl substituiert sind; und entweder p und q beide für 1 stehen oder p 0 ist und q 0 oder 1 darstell; wobei Phenyl in Phenylresten per se und in Phenylniederalkyl-, Phenylniederalkenyl-, Phenylniederalkinyl-, Benzoyl-, Phenoxy- und Benzyloxy-Gruppen jeweils unsubstituiertes oder ein- oder unabhängig voneinander mehrfach durch (einen) Substituenten ausgewählt aus der Gruppe, die aus Halogen, Hydroxy, Niederalkoxy, Niederalkyl und Trifluormethyl besteht, substituiertes Phenyl bedeutet; in freier Form oder in Salzform.

3. Verfahren nach Anspruch 1 zur Herstellung einer Verbindung der Formel I, worin $R_1$ Niederalkyl, Niederalkenyl oder Cycloniederalkyl ist; $R_2$ 1H-Tetrazol-5-yl, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, Amino, durch Niederalkyl, Niederalkenyl, Niederalkinyl oder Phenyl-niederalkyl, -niederalkenyl oder -niederalkinyl mono- oder unabhängig voneinander disubstituiertes oder durch Niederalkylen oder Niederalkylenoxyniederalkylen disubstituiertes Amino, durch Niederalkanoyl oder Benzoyl monosubstituiertes Amino, Formyl, Diniederalkoxymethyl, 1,3-Dioxacycloalk-2-yl, Hydroxy, Niederalkoxy, Niederalkenyloxy, Phenoxy, Benzyloxy, $S(O)_m$-R, wobei m 0, 1 oder 2 ist und R Wasserstoff, Niederalkyl, Niederalkenyl oder Niederalkinyl ist, Niederalkanoyl, Aminosulfonyl, Aminosulfonyl, in welchem die Aminogruppe durch Niederalkyl, Niederalkenyl, Niederalkinyl oder Phenyl-niederalkyl, -niederalkenyl oder -niederalkinyl mono- oder unabhängig voneinander disubstituiert oder durch Niederalkylen oder Niederalkylenoxyniederalkylen disubstituiert ist, $PO_2H_2$, $PO_3H_2$ oder Sulfonylamino, in welchem das Schwefelatom der Sulfonylaminogruppe durch Niederalkyl, Niederalkenyl, Niederalkinyl, Halogen-niederalkyl, -niederalkenyl oder -niederalkinyl, Phenyl-niederalkyl, -niederalkenyl oder -niederalkinyl oder Phenyl monosubstituiert ist, bedeutet; $R_3$ Halogenniederalkansulfonylamino, Carboxy oder 1H-Tetrazol-5-yl ist; X geradkettiges Niederalkylen, geradkettiges Niederalkylen, in welchem eine Methylengruppe durch Cycloniederalk-1,1-ylen ersetzt ist, geradkettiges Niederalkylen, das ein- oder unabhängig voneinander zwei-, drei- oder vierfach durch Niederalkyl, Niederalkenyl oder Niederalkinyl substituiert ist, oder geradkettiges Niederalkylen, das ein- oder unabhängig voneinander zweifach durch Niederalkyl, Niederalkenyl oder Niederalkinyl substituiert ist und in welchem eine Methylengruppe der in der entsprechenden unsubstituierten geradkettigen Niederalkylengruppe vorhandenen Methylengruppen durch Cycloniederalk-1,1-ylen ersetzt ist, darstellt, die Ringe A und B unabhängig voneinander unsubstituiert oder durch Halogen, Hydroxy, Niederalkoxy, $S(O)_m$-R, wobei m 0, 1 oder 2 ist und R Wasserstoff oder Niederalkyl ist, Niederalkyl, Halogen-niederalkyl, Hydroxy-niederalkyl, gegebenenfalls durch Halogen oder Hydroxy substituiertes Niederalkoxy-niederalkyl oder gegebenenfalls durch Halogen oder Hydroxy substituiertes Niederalkenyloxy-niederalkyl substituiert sind; und entweder p und q beide für 1 stehen oder p 0 ist und q 0 oder 1 darstellt; wobei Phenyl in Phenylresten per se und in Phenylniederalkyl-, Phenylniederalkenyl-, Phenylniederalkinyl-, Benzoyl-, Phenoxyund Benzyloxy-Gruppen jeweils unsubstituiertes oder ein- oder unabhängig voneinander zweifach durch (einen) Substituenten ausgewählt aus der Gruppe, die aus Halogen, Hydroxy, Niederalkoxy, Niederalkyl und Trifluormethyl besteht, substituiertes Phenyl bedeutet; in freier Form oder in Salzform.

4. Verfahren gemäss Anspruch 1 zur Herstellung einer Verbindung der Formel I, worin $R_1$ Niederalkyl oder Niederalkenyl ist; $R_2$ 1H-Tetrazol-5-yl, Carboxy, Carboxy, welches durch einen Alkohol verestert ist, dessen alkoholische Hydroxyfunktion an Niederalkyl oder Niederalkoxy-niederalkyl gebunden ist, Carbamoyl, dessen Aminogruppe unsubstituiert oder durch Niederalkyl oder Phenyl-niederalkyl mono- oder unabhängig voneinander disubstituiert oder durch Niederalkylen oder Niederalkylenoxyniederalkylen disubstituiert ist, Amino, durch Niederalkyl oder Phenyl-niederalkyl mono- oder unabhängig voneinander disubstituiertes oder durch Niederalkylen oder Niederalkylenoxyniederalkylen disubstituiertes Amino, durch Niederalkanoyl monosubstituiertes Amino, Hydroxy, Niederalkoxy, Phenoxy, Benzyloxy, $S(O)_m$-R, wobei m 0, 1 oder 2 ist und R Wasserstoff oder Niederalkyl ist, Niederalkanoyl, Aminosulfonyl, $PO_2H_2$, $PO_3H_2$ oder Sulfonylamino, in welchem das Schwefelatom der Sulfonylaminogruppe durch Niederalkyl, Halogenniederalkyl, Phenylniederalkyl oder Phenyl monosubstituiert ist, bedeutet; $R_3$ Carboxy oder 1H-Tetrazol-5-yl ist; X geradkettiges Niederalkylen, geradkettiges Niederalkylen, in welchem eine Methylengruppe durch Cycloniederalk-1,1-ylen ersetzt ist, oder geradkettiges Niederalkylen, das ein- oder unahhängig voneinander zweifach durch Niederalkyl substituiert ist, darstellt; die Ringe A und B unabhängig voneinander unsubstituiert oder durch Halogen, Hydroxy, Niederalkoxy, $S(O)_m$-R, wobei m 0, 1 oder 2 ist und R Wasserstoff oder Niederalkyl ist, oder Niederalkyl substituiert sind; p 0 ist, q 0 oder 1 darstellt; und der Ring B in 4-Position des Ringes A gebunden ist; wobei Phenyl in Phenylniederalkyl-, Phenoxy- und Benzyloxy-Gruppen jeweils unsubstituiertes oder einfach durch einen Substituenten ausgewählt aus der Gruppe, die aus Halogen, Niederalkoxy, Niederalkyl und Trifluormethyl besteht, substituiertes Phenyl bedeutet; in freier Form oder in Salzform.

5. Verfahren gemäss Anspruch 1 zur Herstellung einer Verbindung der Formel I, worin X für das Strukturelement der Formel -$X_1$-Ph-$X_2$- steht, wobei $X_1$ und $X_2$ unabhängig voneinander für eine Bindung oder für $C_1$-$C_7$-Alklyen stehen und Ph Phenylen bedeutet; wobei Phenylen unsubstituiertes oder einfach durch einen Substituenten ausgewählt aus der Gruppe, die aus Halogen, Niederalkoxy, Niederalkyl und Trifluormethyl besteht, substituiertes Phenylen bedeutet; in freier Form oder in Salzform.

6. Verfahren gemäss Anspruch 1 zur Herstellung einer Verbindung der Formel I, worin der Ring B in 4-Position des Ringes A gebunden ist; wobei Phenyl in Phenylniederalkyl-, Phenoxy- und Benzyloxy-Gruppen

jeweils unsubstituiertes oder einfach durch einen Substituenten ausgewählt aus der Gruppe, die aus Halogen, Niederalkoxy, Niederalkyl und Trifluormethyl besteht, substituiertes Phenyl bedeutet; in freier Form oder in Salzform.

7. Verfahren gemäss Anspruch 1 zur Herstellung einer Verbindung der Formel I, worin $R_1$ $C_1$-$C_4$-Alkyl oder $C_3$-$C_5$-Alkenyl ist, $R_2$ 1H-Tetrazol-5-yl, Carboxy, $C_1$-$C_4$-Alkoxycarbonyl, Carbamoyl, N-$C_1$-$C_4$-Alkylcarbamoyl, N,N-Di-$C_1$-$C_4$-alkylcarbamoyl, $C_1$-$C_4$-Alkanoyl, Aminosulfonyl oder Sulfonylamino, in welchem das Schwefelatom der Sulfonylaminogruppe durch $C_1$-$C_4$-Alkyl monosubstituiert ist, bedeutet; $R_3$ Carboxy oder 1H-Tetrazol-5-yl ist; X geradkettiges $C_1$-$C_7$-Alkylen, in welchem eine Methylengruppe durch $C_3$-$C_7$-Cycloalk-1,1-ylen ersetzt ist, oder geradkettiges $C_1$-$C_7$-Alkylen, das ein- oder unabhängig voneinander zweifach durch $C_1$-$C_4$-Alkyl substituiert ist, darstellt; die Ringe A und B unsubstituiert sind, p 0 ist, q 1 darstellt; der Ring B in 4-Position des Ringes A gebunden ist; und $R_3$ in einer 2-Position des Ringes B gebunden ist; in freier Form oder in Salzform.

8. Verfahren gemäss Anspruch 1 zur Herstellung einer Verbindung der Formel I, worin $R_1$ $C_1$-$C_4$-Alkyl oder $C_3$-$C_5$-Alkenyl ist, $R_2$ 1H-Tetrazol-5-yl, Carboxy, $C_1$-$C_4$-Alkoxycarbonyl, Carbamoyl, N-$C_1$-$C_4$-Alkylcarbamoyl, N,N-Di-$C_1$-$C_4$-alkylcarbamoyl, $C_1$-$C_4$-Alkanoyl, Aminosulfonyl oder Sulfonylamino, in welchem das Schwefelatom der Sulfonylaminogruppe durch $C_1$-$C_4$-Alkyl monosubstituiert ist, bedeutet; $R_3$ Carboxy oder 1H-Tetrazol-5-yl ist; X für das Strukturelement der Formel -$X_1$-Ph-$X_2$- steht, wobei $X_1$ und $X_2$ unabhängig voneinander für eine Bindung oder für $C_1$-$C_3$-Alklyen stehen und Ph Phenylen bedeutet; die Ringe A und B unsubstituiert sind, p 0 ist, q 1 darstellt; der Ring B in 4-Position des Ringes A gebunden ist; und $R_3$ in einer 2-Position des Ringes B gebunden ist; in freier Form oder in Salzform.

9. Verfahren gemäss Anspruch 1 zur Herstellung einer Verbindung der Formel I, worin $R_1$ $C_1$-$C_4$-Alkyl ist, $R_2$ Carboxy, $C_1$-$C_4$-Alkoxycarbonyl oder Carbamoyl bedeutet, $R_3$ 1H-Tetrazol-5-yl ist, X geradkettiges $C_1$-$C_7$-Alkylen, in welchem eine Methylengruppe durch $C_3$-$C_7$-Cycloalk-1,1-ylen ersetzt ist, oder geradkettiges $C_1$-$C_7$-Alkylen, das ein- oder unabhängig voneinander zweifach durch $C_1$-$C_4$-Alkyl substituiert ist, darstellt, die Ringe A und B unsubstituiert sind, p 0 ist, q 1 darstellt, der Ring B in 4-Position des Ringes A gebunden ist und $R_3$ in einer 2-Position des Ringes B gebunden ist, in freier Form oder in Salzform.

10. Verfahren gemäss Anspruch 1 zur Herstellung einer Verbindung der Formel I, worin $R_1$ $C_1$-$C_4$-Alkyl ist, $R_2$ Carboxy bedeutet, $R_3$ 1H-Tetrazol-5-yl ist, X geradkettiges $C_1$-$C_7$-Alkylen, in welchem eine Methylengruppe durch $C_3$-$C_7$-Cycloalk-1,1-ylen ersetzt ist, oder geradkettiges $C_1$-$C_7$-Alkylen, das ein- oder unabhängig voneinander zweifach durch $C_1$-$C_4$-Alkyl substituiert ist, darstellt, die Ringe A und B unsubstituiert sind, p 0 ist, q 1 darstellt, der Ring B in 4-Position des Ringes A gebunden ist und $R_3$ in einer 2-Position des Ringes B gebunden ist, in freier Form oder in Salzform.

11. Verfahren gemäss Anspruch 1 zur Herstellung einer Verbindung der Formel I, worin $R_1$ $C_3$-$C_4$-Alkyl ist; $R_2$ Carboxy bedeutet; $R_3$ 1H-Tetrazol-5-yl ist; X 2-Methyl-but- 1,3-ylen ist oder 1,3-Propylen bedeutet, in welchem die ω-Methylengruppe durch Cyclohex-1,1-ylen ersetzt ist; die Ringe A und B unsubstituiert sind; p 0 ist; q 1 darstellt; der Ring B in 4-Position des Ringes A gebunden ist; und $R_3$ in einer 2-Position des Ringes B gebunden ist; in freier Form oder in Salzform.

12. Verfahren gemäss Anspruch 1 zur Herstellung einer Verbindung ausgewählt aus
3-Carboxypropansäure-N-butyl-N-[2′-(1H-tetrazol-5-yl)biphenyl-4-ylmethyl]-amid, in freier Form oder in Salzform;
3-Carboxy-3-methyl-butansäure-N-butyl-N-[2′-(1H-tetrazol-5-yl)biphenyl-4-ylmethyl]amid, in freier Form oder in Salztorm;
4-Carboxy-4-methyl-pentansäure-N-butyl-N-[2′-(1H-tetrazol-5-yl)biphenyl-4-ylmethyl]amid, in freier Form oder in Salzform;
4-Carboxy-3,3-dimethyl-butansäure-N-propyl-N-[2′-(1H-tetrazol-5-yl)biphenyl-4-ylmethyl]-amid, in freier Form oder in Salzform;
4-Methoxycarbonyl-4-methyl-pentansäure-N-propyl-N-[2′-(1H-tetrazol-5-yl)biphenyl-4-ylmethyl]-amid, in freier Form oder in Salzform;
4-Carboxy-4-methyl-pentansäure-N-propyl-N-[2′-(1H-tetrazol-5-yl)biphenyl-4-ylmethyl]-amid, in freier Form oder in Salzform;
4-Carboxy-4-ethyl-hexansäure-N-butyl-N-[2′-(1H-tetrazol-5-yl)biphenyl-4-ylmethyl]amid, in freier Form oder in Salzform;

2-Carboxybutansäure-N-butyl-N-[2′-(1H-tetrazol-5-yl)biphenyl-4-ylmethyl]-amid, in freier Form oder in Salzform;
4-Carboxy-4-ethyl-hexansäure-N-propyl-N-[2′-(1H-tetrazol-5-yl)biphenyl-4-ylmethyl]amid, in freier Form oder in Salzform;
4-Carboxy-3,3-tetramethylen-butansäure-N-propyl-N-[2′-(1H-tetrazol-5-yl)biphenyl-4-ylmethyl]-amid, in freier Form oder in Salzform;
4-Carboxy-3,3-tetramethylen-butansäure-N-butyl-N-[2′-(1H-tetrazol-5-yl)biphenyl-4-ylmethyl]-amid, in freier Form oder in Salzform;
3-Carboxy-3,3-tetramethylen-propansäure-N-propyl-N-[2′-(1H-tetrazol-5-yl)biphenyl-4-ylmethyl]-amid, in freier Form oder in Salzform;
3-Carboxy-3,3-tetramethylen-propansäure-N-butyl-N-[2′-(1H-tetrazol-5-yl)biphenyl-4-ylmethyl]-amid, in freier Form oder in Salzform;
3-Carboxy-3-ethyl-pentansäure-N-propyl-N-[2′-(1H-tetrazol-5-yl)biphenyl-4-ylmethyl]amid, in freier Form oder in Salzform;
4-Carboxy-4,4-tetramethylen-butansäure-N-propyl-N-[2′-(1H-tetrazol-5-yl)biphenyl-4-ylmethyl]-amid, in freier Form oder in Salzform;
4-Carboxy-4,4-tetramethylen-butansäure-N-butyl-N-[2′-(1H-tetrazol-5-yl)biphenyl-4-ylmethyl]-amid, in freier Form oder in Salzform;
2-Carboxy-2-ethyl-butansäure-N-butyl-N-[2′-(1H-tetrazol-5-yl)biphenyl-4-ylmethyl]amid, in freier Form oder in Salzform;
2-Carboxy-2-ethyl-butansäure-N-propyl-N-[2′-(1H-tetrazol-5-yl)biphenyl-4-ylmethyl]amid, in freier Form oder in Salzform;
2-Carboxybutansäure-N-propyl-N-[2′-(1H-tetrazol-5-yl)biphenyl-4-ylmethyl]-amid, in freier Form oder in Salzform;
3-Carboxy-3-ethyl-pentansäure-N-butyl-N-[2′-(1H-tetrazol-5-yl)biphenyl-4-ylmethyl]amid, in freier Form oder in Salzform;
3-Carboxy-3,3-pentamethylen-propansäure-N-propyl-N-[2′-(1H-tetrazol-5-yl)biphenyl-4-ylmethyl]-amid, in freier Form oder in Salzform;
3-Carboxy-3,3-pentamethylen-propansäure-N-butyl-N-[2′-(1H-tetrazol-5-yl)biphenyl-4-ylmethyl]-amid, in freier Form oder in Salzform;
5-Carboxy-5,5-tetramethylen-pentansäure-N-propyl-N-[2′-(1H-tetrazol-5-yl)biphenyl-4-ylmethyl]-amid, in freier Form oder in Salzform;
5-Carboxy-5,5-tetramethylen-pentansäure-N-butyl-N-[2′-(1H-tetrazol-5-yl)biphenyl-4-ylmethyl]-amid, in freier Form oder in Salzform;
3-Carboxy-2,2-tetramethylen-propansäure-N-propyl-N-[2′-(1H-tetrazol-5-yl)biphenyl-4-ylmethyl]-amid, in freier Form oder in Salzform;
3-Carboxy-2,2-tetramethylen-propansäure-N-butyl-N-[2′-(1H-tetrazol-5-yl)biphenyl-4-ylmethyl]-amid, in freier Form oder in Salzform;
2,2-Pentamethylen-2-trifluormethansulfonylamino-ethansäure-N-butyl-N-[2′-(1H-tetrazol-5-yl)biphenyl-4-ylmethyl]-amid,in freier Form oder in Salztorm; und
2,2-Pentamethylen-2-trifluormethansulfonylamino-ethansäure-N-propyl-N-[2′-(1H-tetrazol-5-yl)biphenyl -4-ylmethyl]-amid, in freier Form oder in Salzform.

**13.** Verfahren gemäss Anspruch 1 zur Herstellung einer Verbindung ausgewählt aus
4-Carboxy-4,4-pentamethylen-butansäure-N-propyl-N-[2′-(1H-tetrazol-5-yl)-biphenyl-4-ylmethyl] -amid;
4-Carboxy-4,4-pentamethylen-butansäure-N-butyl-N-[2′-(1H-tetrazol-5-yl)-biphenyl-4-ylmethyl]-amid;
N-Propyl-N-[2′-(1H-tetrazol-5-yl)-biphenyl-4-ylmethyl]-isophthalsäure-mono-amid;
2-Carboxy-phenylessigsäure-N-butyl-N-[2′-(1H-tetrazol-5-yl)-biphenyl-4-ylmethyl]amid;
N-Butyl-3-methyl-N-[2′-(1H-tetrazol-5-yl)biphenyl-4-ylmethyl]-2-(S)-trifluormethansulfonylamino-butans äureamid; und
2-(S)-Methansulfonylamino-3-methyl-butansäure-N-butyl-N-[2′-(1H-tetrazol-5-yl)biphenyl-4-ylmethyl]-amid, jeweils in freier Form oder in Salzform.

**14.** Verfahren zur Herstellung eines pharmazeutischen Präparats, dadurch gekennzeichnet, dass man einen Wirkstoff der Formel I gemäss einem der Ansprüche 1-13, in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

**15.** Verwendung einer Verbindung gemäss einem der Ansprüche 1 bis 13, in freier Form oder in Form eines pharmazeutisch verwendbaren Salzes, zur Herstellung eines pharmazeutischen Präparats zur Behandlung von Bluthochdruck und/oder Herzinsuffizienz.